# EUROPEAN PATENT SPECIFICATION

(11) **EP 0 845 976 B1**
(45) Date of publication and mention of the grant of the patent: **24.03.2004**
(21) Application number: 96926802.8
(22) Date of filing: 02.08.1996
(51) Int. Cl.: A61K 7/16, A61K 7/18, A61K 9/68, A61K 33/24, B65D 35/22

(54) **REMINERALIZING PRODUCTS AND METHODS FOR TEETH**
MITTEL UND VERFAHREN ZUR REMINERALISIERUNG DER ZÄHNE
PRODUITS ET PROCEDES DE REMINERALISATION DES DENTS

(30) Priority: 08.08.1995 US 512286; 08.08.1995 US 512473
(43) Date of publication of application: 10.06.1998
(73) Proprietor: Church & Dwight Co., Inc., Princeton, New Jersey 08543 (US)
(72) Inventor: WINSTON, Anthony, E., East Brunswick, NJ 08816 (US); USEN, Norman, Marlboro, NJ 07746 (US)
(74) Representative: Lee, Nicholas John
(86) International application number: PCT/US1996/012456
(87) International publication number: WO 1997/006774

(56) References cited:
- US-A- 3 747 804
- US-A- 4 083 955
- US-A- 4 177 258
- US-A- 4 183 915
- US-A- 4 233 288
- US-A- 4 348 381
- US-A- 4 397 837
- US-A- 4 424 203
- US-A- 4 436 720
- US-A- 4 487 757
- US-A- 4 610 873

## Description

### BACKGROUND OF THE INVENTION

This invention relates to improved products and methods for remineralizing subsurface lesions in teeth. More specifically, this invention relates to stable one-part and two-part products containing water-soluble calcium and phosphate salts which when applied to lesions in dental enamel result in remineralization of subsurface dental enamel and/or mineralization of tubules in exposed dentin, thereby counteracting caries and/or hypersensitivity. The soluble salts may be contained in toothpastes, gels, mouthwashes, wafers, lozenges, chewing gums, food products, and the like, using both single-vehicle systems, e.g., a stable dry blend or a stable solution, or two-vehicle systems, e.g., two dry salts separated by a barrier or two solutions wherein one contains calcium ions and the other phosphate ions.

The primary component of the enamel and dentin in teeth is calcium phosphate in the form of calcium hydroxyapatite. This material is highly insoluble at normal oral pHs. However, carious lesions form in teeth when they are subjected to acids produced from the glycolysis of sugars by the action of various oral bacteria. This is because calcium phosphate salts are more soluble in acidic media.

Saliva is supersaturated with respect to calcium and phosphate ions. Saliva therefore helps protect teeth against demineralization and can slowly remineralize teeth which have become demineralized by acids. It is well known that the presence of fluoride ions can enhance the natural remineralization process and this is one of the accepted mechanisms by which fluoride toothpastes and rinses protect against caries. The efficacy of fluoride-containing toothpastes and rinses to remineralize teeth is limited by the modest levels of calcium and phosphate in saliva. It is evident from the prior art that it is highly desirable to increase the available concentration of calcium and phosphate ions in the oral cavity to speed up the remineralization process. However, because of calcium phosphate's low solubility at the pH of saliva, the addition of higher levels of dissolved calcium and phosphate ions is not easily accomplished.

Remineralization of dental enamel has been carried out experimentally, both in vivo and in vitro. Some studies have concentrated on the remineralizing properties of saliva and synthetic solutions supersaturated with respect to hydroxyapatite. Such studies comprise the subject matter of U.S. Patent Nos. 3,679,360 (Rubin) and 4,097,935 (Jarcho).

Generally, the supersaturated solutions or slurries used in these patents for remineralization experiments have been prepared from a single form of calcium phosphate. When a carious lesion is flooded with one of these supersaturated solutions, the calcium and phosphate ions remineralize the lesion by forming precipitated hydroxyapatite.

However, these solutions are impractical for use for several reasons. First, the amount of calcium and phosphate ions available for remineralization in these supersaturated solutions is too low. It is reported that it takes approximately 10,000 unit volumes of the usual supersaturated solution to produce one unit volume of mineral. Thus, remineralization by this method requires both an excessive volume of fluid and an excessive number of applications. The supersaturated solutions are inherently limited in this respect because such solutions cannot maintain their supersaturated state. When the hydroxyapatite precipitates out to the point where the solution is no longer supersaturated, new supersaturated solution must be introduced or the remineralization process stops.

Another problem with single calcium phosphate slurries is that as the hydroxyapatite precipitates out of solution, the pH of the solution changes. Unless the old solution is removed from contact with the tooth material, the solution may become too acidic or alkaline and damage the dental tissue.

U.S. Patent No. 4,080,440 (Digiulio et al) discloses a metastable solution of calcium and phosphate ions at a low pH (between 2.5 and 4.0) under which conditions the solubility of calcium phosphate salts is high. After penetration of the solution into demineralized enamel, remineralization results from the precipitation of calcium phosphate salts when the pH rises. Fluoride ions can be included in the metastable solution. A significant disadvantage of the use of metastable solutions is that the relatively low pH might demineralize the dental enamel and/or injure other tissue.

U.S. Patent Nos. 4,177,258, 4,183,915 and 4,348,381 (Gaffar et al) provide for a remineralizing solution containing supersaturated concentrations of calcium ions, phosphate ions and a fluoride source stabilized by the presence of an antinucleating agent such as diamine tetramethylenephosphonic acid, ethylenediamine tetramethylenephosphonic acid and 2-phosphonobutane-tricarboxylic acid-1,2,4, or the water-soluble salts thereof. This solution is preferably adjusted to the neutral pH range where it is alleged to most effectively remineralize subsurface lesions. Even though the antinucleating agent would be expected to stabilize the solution, equilibrium of the supersaturated concentrations is still found difficult to maintain and avoid precipitation of hydroxyapatite and changes in the pH of the solution.

U.S. Patent Nos. 4,083,955 (Grabenstetter et al) and 4,397,837 (Raaf et al) provide a process for remineralizing demineralized enamel by the consecutive treatment of tooth surfaces with separate solutions containing calcium ions and phosphate ions. In this process, fluoride ions may be present in the phosphate solutions. It is immaterial which ionic solution is used to treat the teeth first. By sequentially applying calcium and phosphate ions to the tooth surface, high concentrations of the ions are able to penetrate into lesions in solution form, where they precipitate as a calcium phosphate salt when ions from the second treatment solution diffuse in. While apparently successful, this method involves the inconvenience of employing two different chewing portions, one containing the calcium ions and the other the phosphate ions. This method could be confusing because of the necessity of ensuring the proper sequence of gum portions and also inconvenient due to the plurality of sequential applications which can be found to be time-consuming.

U.S. Patent No. 4,606,912 (Rudy et al.) provides a method of making a clear aqueous mouthwash solution capable of remineralizing lesions in teeth by forming an aqueous solution containing a source of calcium ions and a chelating agent for calcium ions, causing the chelation of at least 50% of the calcium ions and subsequently adding a source of phosphate ions to the aqueous solution. Here too while somewhat effective, the addition and necessary control of the amount of chelating agent makes the concept impractical.

Another problem with known remineralization techniques is that the remineralization may stop before the lesion is completely remineralized due to build-up of the remineralized tooth material in or on the outer layer of the tooth's surface. This build-up occurs when the rate of remineralization is too fast and prevents the diffusion of the mineral into the deeper regions of the lesion, thus thwarting the full remineralization of the tooth.

U.S. Patent Nos. 5,037,639; 5,268,167; 5,437,857; 5,427,768; and 5,460,803 (all to Tung) involve the use of amorphous calcium compounds such as amorphous calcium phosphate (ACP), amorphous calcium phosphate fluoride (ACPF) and amorphous calcium carbonate phosphate (ACCP) for use in remineralizing teeth. These amorphous compounds or solutions which form the amorphous compounds when applied either onto or into dental tissue prevent and/or repair dental weaknesses such as dental caries, exposed roots and dentin sensitivity. The compounds are claimed to have high solubilities, fast formation rates and fast conversion rates (to apatite).

Remineralization is accomplished by bringing the amorphous compound into contact with the dental tissue. This can be done directly, i.e., putting an amorphous compound directly on the tooth, or indirectly through a carrier, i.e., incorporating the amorphous compound in a carrier such as a gel, a chewing gum, or a toothpaste and applying the carrier to the dental tissue. Once contact is established with the tooth, the amorphous calcium phosphate compounds will recrystallize to the less soluble apatite form in the lesion and reform the tooth. However, under conditions where amorphous calcium phosphate compounds are stable, the quantity of calcium and phosphate released is relatively low and, therefore, remineralization is slower than desirable.

The aforementioned patents to Tung also teach the use of two-part solutions wherein a first part contains phosphate salt(s) and a second part contains calcium salt(s), wherein either the first part or the second part further contains carbonate salt(s). In addition, the Tung patents teach solutions formed by dissolving in water a solid powder containing calcium salt(s), phosphate salt(s), and carbonate salt(s). These solutions are then applied to dental tissue. The Tung patents further teach the use of non-carbonated solid powders containing mixtures of calcium salts and phosphate salts which can be applied directly to the tooth or dispersed in gel, chewing gum, or other non-aqueous mediums such as toothpaste which is placed in contact with the tooth. The patents teach that these powders are easily dissolved in saliva and then reprecipitated as an amorphous calcium phosphate compound. However, the Tung patents do not disclose the pHs of aqueous solutions formed from the non-carbonated solid powder.

It would be desirable to provide effective remineralizing/mineralizing products and methods which do not require the presence of carbonate salts to achieve stability, remineralization and/or mineralization. It would be further desirable to provide remineralizing/mineralizing products and methods which directly form hydroxyapatite at the subsurface of the tooth rather than first forming an amorphous calcium phosphate as an intermediate.

International Patent WO 94/18938 (Greenberg) adds calcium glycerophosphate to a chewing gum. The calcium glycerophosphate is said to increase calcium and phosphate concentrations in plaque, increasing remineralization and decreasing demineralization. It may also inhibit large drops in pH in plaque and interfere with metabolism of S. mutans. A problem with this technology is that the glycerophosphate ion has to hydrolyze and release free phosphate ions before it can participate in the remineralization process. Hydrolysis occurs in the mouth due to the presence of phosphatase enzymes. However, the process is slow. The high concentration of calcium ions supplied by the calcium glycerophosphatase therefore have time to dissipate before sufficient phosphate can be released to produce maximum remineralization.

Chewing gums which may contain calcium and phosphate salts are also disclosed in U.S. Patent No. 4,233,288 (Cornell).

Thus, one problem with known remineralization compositions and techniques is that there is not a one-part, stable remineralizing composition that may be suitably prepared as a chewing gum which is not negatively affected by a rise in pH or temperatures or which can efficiently remineralize teeth.

Thus, there is a need for a method of remineralizing dental enamel which employs a stable, one-part remineralizing product which does not require excessive amounts of calcium and phosphate salts or inordinately long, frequent or sequential exposure times.

For mineralization or remineralization of enamel or dentin to occur, the concentration of calcium and phosphate ions in the saliva must be above the concentration required to saturate the solution with respect to the formation of calcium hydroxyapatite, octacalcium phosphate, dicalcium phosphate dihydrate, or other form of insoluble calcium phosphate. At pHs above about 6.5, these requirements are met by the levels of calcium and phosphate found in normal human saliva. However, because the concentration of calcium and phosphate ions in normal human saliva is low, even at pHs above 6.5, the rate of mineralization produced by normal saliva is very slow even when fluoride is present to catalyze the process. However, when the pH of the saliva is above about 7, increasing the concentration levels of calcium and phosphate ions much beyond that normally present in saliva does not significantly increase remineralization. Because calcium phosphates are highly insoluble at pH levels above about 7, excessively rapid precipitation occurs which does not allow time for the ions to penetrate the tooth.

At pHs below about 7, significant remineralization will occur only if the concentration of calcium and phosphate ions in the saliva is above the concentration required to saturate the solution with respect to the formation of dicalcium phosphate dihydrate or calcium hydroxyapatite. Under these pH conditions, remineralization can be accelerated by increasing the degree of supersaturation in saliva. Inasmuch as the solubility of these phosphates increases with decreasing pH, it has been found that when lesions are remineralized with solutions having a pH in the range of 4.5 to 7.0 and containing supersaturated quantities of calcium and phosphate ions, the optimum concentration of calcium ions needed to maximize the process increases with decreasing pH. Below a pH of about 4.0, dicalcium phosphate dihydrate becomes the stable precipitating species from supersaturated solutions. Under these pH conditions, it takes very high levels of calcium and phosphate to saturate the solutions. Under such conditions, there is a real danger with fairly high concentrations of calcium and phosphate that the solution will be undersaturated and demineralization of the teeth being treated will occur.

Accordingly, it is a primary object of this invention to provide products and methods for the remineralization and the prevention of demineralization of human teeth, wherein the products and methods are capable of effectively incorporating calcium ions, phosphate ions, and, if desired, fluoride ions into the dental enamel, the products also being easily usable by the consumer and not differing significantly, in flavor and appearance, from customary dental cosmetics.

It is another object of this invention to provide an improved product and a method of preparing such product, wherein the product is maintainable in a single container, substrate or matrix and is capable of remineralizing lesions in the teeth and mineralizing normal teeth to prevent cariogenic lesions from forming.

It is an additional object of the present invention to provide a single-part stable chewing gum product which is capable of remineralizing lesions in teeth and mineralizing normal teeth to prevent cariogenic lesions from forming.

It is a further object of this invention to provide products and methods for the remineralization and the prevention of demineralization of human teeth, which substantially prevent precipitation of the calcium and phosphate ions prior to diffusion of the ions into the subsurface of the tooth without reducing the rate of remineralization at the subsurface of the tooth.

It is an additional object of this invention to provide effective remineralizing/mineralizing products and methods wherein the products are non-carbonated.

It is a further object of this invention to provide remineralizing/mineralizing products and methods which can directly form hydroxyapatite in the subsurface of a tooth subsurface without first forming an amorphous calcium phosphate as an intermediate.

It is a further object of this invention to provide two-part remineralizing/mineralizing products and methods of using same, wherein the products contain calcium salt(s) in a first part and phosphate salt(s) in a second part separate from the first part prior to introduction of the product into the oral cavity but wherein the product will simultaneously dispense the first and second parts from the product for use in the oral cavity.

Another object of the present invention is to provide products having the characteristics set forth in the foregoing objects and which are in the form of a toothpaste, gel, professional gel, mouthwash, mouthrinse, chewing gum, food product, and the like.

A further object of the present invention is to provide remineralization/mineralization methods using products having the characteristics set forth in the preceding objects.

These and other objects which are achieved according to the present invention can be readily discerned from the following description.

### SUMMARY OF THE INVENTION

In the present invention, the aforementioned objects are achieved by applying to the teeth a one-part or two-part product which contains remineralization components which do not react to any large extent until introduced into the oral cavity and upon introduction do not rapidly precipitate. The one-part product contains at least one water-soluble calcium compound, at least one other water-soluble, non-toxic compound containing a divalent metal different from calcium, and at least one water-soluble phosphate compound. If desired, at least one water-soluble fluorine compound may be added to the system. In two-part products, one part contains the calcium and divalent metal compounds and the other part contains the phosphate compound and optionally, the fluoride compound. In this way, the ions which effect remineralization can be absorbed by the dental enamel and their subsequent, but controlled, reaction causes rehardening of demineralized areas in the dental enamel.

It has been found that effective remineralizing treatments can be prepared by providing stable (if desired, non-aqueous) products composed of soluble salts containing high concentrations of calcium, phosphate and, if desired, fluoride ions and applying the products to the teeth at moderate pHs. However, the calcium ions must be prevented from reacting with the phosphate ions or fluoride ions until immediately before use (e.g., in the case of chewing gum products, until mastication begins) and then preferably prevented from rapidly precipitating so as to allow ample time for diffusion of calcium and phosphate ions into the teeth. This can be accomplished by utilizing, in addition to the calcium compound, the aforementioned water-soluble, non-toxic divalent metal salt wherein the divalent metal is other than calcium. The divalent metal is preferably a metal selected from the group consisting of magnesium, strontium, tin and zinc. Magnesium is the most preferred divalent metal.

It is an advantage of the present invention that the one-part remineralization product can be packaged or otherwise contained as a stable dry-mix which can be subsequently suspended or dissolved in water to form a paste product or the like or a mouthwash, respectively, or added to a gum base to form a chewing gum product. The dry-mix product may be in the form of a powder, granular material, flake or the like. The dry-mix product contains from 1.0% to 80.0% of the at least one calcium salt, from 1.0% to 80.0% of the at least one phosphate salt, from 0.1% to 20.0% of the at least one water-soluble divalent metal salt, wherein the metal is other than calcium, from 0.1% to 20.0% of flavor, from 0.1% to 30.0% of sweetener, from 0 to 10.0% of a fluoride salt, and from 0 to 5.0% of a surfactant. The flavor is preferably provided as a spray dried powder.

It is another advantage of the present invention that one-part stable remineralizing products may be suitably prepared as, for example, toothpastes, gels, professional gels, i.e., those which are applied professionally or are obtained by a prescription, mouthwashes, mouthrinses, chewing gums, powders, and edible solid products, e.g., lozenges, candies, foods and the like.

It is still another advantage that such products may be made effervescent when added to water by including therein a non-toxic organic acid, e.g., malic acid and an alkali-metal bicarbonate, e.g., sodium bicarbonate. However, it is to be understood that the products of this invention do not require the presence of carbonate salts to achieve stability, remineralization and/or mineralization. Thus, the products of this invention may be non-carbonated.

In one embodiment thereof, the present invention is directed to a stable one-part product for effecting remineralization of one or more lesions formed in at least one subsurface of at least one tooth and/or mineralization of one or more tubules in exposed dentin. This product, which can be aqueous or non-aqueous and either carbonated or non-carbonated, contains:
(1) from 0.05% to 15.0%, preferably from 0.10% to 10.0%, by weight of at least one water-soluble calcium salt;
(2) at least 0.001%, preferably from 0.001% to 2.0%, by weight of at least one water-soluble, non-toxic divalent metal salt other than a calcium salt;
(3) from 0.05% to 15.0%, preferably from 0.10% to 10.0%, by weight of at least one water-soluble phosphate salt; and
(4) from 0% to 5.0%, preferably from 0.01% to 5.0% by weight of at least one water-soluble fluoride salt;
wherein when ingredients (1)-(4) are mixed with water to form an aqueous mixed solution, the solution has a pH of from 4.5 to 7.0.

The one-part product described hereinabove usually contains acidic or basic compounds which provide that the pH is between 4.5 and 7.0, preferably between 5.0 and 7.0, most preferably between 5.0 and 5.75 when the dry mix is solubilized or the final aqueous solution is prepared.

A specific product within the scope of the present invention is a stable, non-aqueous, single-part chewing gum. Such chewing gum preferably contains from 0.01% to 15.0% of the calcium salt, greater than 0.0002%, preferably from 0.0002% to 1.0%, of the divalent metal salt, from 0.01% to 15.0% of the phosphate salt, and, if desired, from 0.0001% to 0.5% of the fluoride compound. Such chewing gum compositions may also contain suitable pH adjusting compounds, as described hereinabove.

To effect remineralization and/or mineralization, the chewing gum product of this invention is applied directly to the teeth when chewed and solubilized with saliva.

As mentioned previously herein, the stable, single-part chewing gum product of this invention may be prepared by adding to a gum base the dry-mix concentrated product described hereinabove. The chewing gum product should contain from 0.005% to 20.0%, preferably 0.1% to 7.0%, of the dry mix. Other adjuvants are, of course, included.

Methods of effecting remineralization/mineralization with other products within the scope of the aforementioned one-part embodiment, e.g. mouthwashes, toothpastes, gels and the like, generally involve the steps of:
(A) providing the aforementioned one-part product;
(B) mixing ingredients (1)-(4) together to form the aqueous mixed solution; and
(C) contacting the at least one tooth with the aqueous mixed solution for a sufficient period of time that calcium ions released from the calcium salt(s) and phosphate ions released from the phosphate salt(s) diffuse into the at least one subsurface of the at least one tooth and precipitate to form a calcium phosphate salt at the at least one subsurface and thereby remineralize the one or more lesions and/or penetrate into one or more tubules in exposed dentin and precipitate to form a calcium phosphate salt and thereby mineralize the one or more tubules.

A second embodiment of the present invention is directed to a stable two-part product for effecting remineralization of one or more lesions formed in at least one subsurface of at least one tooth and/or mineralization of one or more tubules in the exposed dentin of at least one tooth. This product, which can be aqueous or non-aqueous and either carbonated or non-carbonated, contains:
(1) a first discrete part containing from 0.05% to 15.0%, preferably from 0.10% to 10.0%, by weight of at least one water-soluble calcium salt and at least 0.001%, preferably from 0.001% to 2.0%, by weight of at least one water-soluble, non-toxic divalent metal salt other than a calcium salt;
(2) a second discrete part containing from 0.05% to 15.0%, preferably from 0.10% to 10.0%, by weight of at least one water-soluble phosphate salt and from 0% to 5.0%, preferably from 0.01% to 5.0%, by weight of at least one water-soluble fluoride salt; and
(3) a dispensing means for simultaneously dispensing the first and second parts; wherein when the simultaneously dispensed first and second parts are mixed to form an aqueous mixed solution, the solution has a pH of from 4.5 to 7.0, preferably from 5.0 to 7.0, more preferably from 5.0 to 5.75.

In using the two-part aqueous product of this invention, the two parts are mixed together to form an aqueous mixed solution and the solution is immediately applied to the teeth being treated. With a two-part non-aqueous product of this invention, the two parts are mixed with one another and with water (e.g., saliva) to form an aqueous mixed solution which is then applied to the teeth being treated.

Remineralizing/mineralizing methods using the aforementioned two-part product generally involves the steps of:
(A) providing the aforementioned two-part product;
(B) simultaneously dispensing the first and second parts;
(C) mixing the simultaneously dispensed first and second parts to form the aqueous mixed solution; and
(D) contacting the at least one tooth with the aqueous mixed solution for a sufficient period of time that calcium ions released from the at least one calcium salt and phosphate ions released from the at least one phosphate salt diffuse into the at least one subsurface of the at least one tooth and precipitate to form hydroxyapatite at the at least one subsurface and thereby remineralize the one or more lesions and/or mineralize the one or more tubules.

The products of this invention effect remineralization of lesions and mineralization of tubules but impede the precipitation of calcium phosphate so as to improve diffusion of calcium and phosphate ions into the teeth.

The products of the present invention give substantially improved remineralization and mineralization as compared with prior art compositions. Fluoride-containing toothpastes within the scope of the present invention are much more effective than conventional fluoride-containing toothpastes in remineralizing teeth, while the non-fluoride products of the present invention are more or less equivalent to conventional fluoride-containing dentifrices.

The disadvantages of the prior art methods are overcome by the present invention which effects subsurface remineralization rather than surface remineralization. Since dental caries begins as a subsurface demineralization of the dental enamel, subsurface remineralization arrests and repairs the carious lesion before any permanent structural damage to the tooth occurs. The present invention does not require preparation of the enamel surface, capping of the tooth, or removal of decay products. Further, the present invention may be conveniently practiced by the public without substantially changing their dental care habits.

### DESCRIPTION OF THE INVENTION

The present invention lies in the discovery that a distinct improvement is realized when teeth are remineralized or desensitized by the application of products, e.g., pastes, gels, solutions, chewing gums, bubble gums, dragees, food products and the like, which contain soluble salts yielding ions which will react to form a desirable remineralizing or desensitizing precipitate. The improvement in the application comprises the simultaneous use of a water-soluble salt of a divalent metal compound, other than calcium, which is admixed with the soluble salts which are placed in contact with the tooth surface. In this reaction, selected cations and anions diffuse through the tooth surface to its demineralized subsurface. However, the additional divalent metal cations contained in the reactant solution stabilize the system from rapidly precipitating the calcium cations and the phosphate anions. The remineralizing cations and anions can then diffuse through the tooth surface to the demineralized subsurface without rapidly forming the precipitate which is bound to the tooth structure. As a result, the tooth's subsurface is more effectively remineralized or desensitized when an effective amount of the divalent metal cations is used.

As discussed above, the products of the invention are optionally non-aqueous. By "non-aqueous" is meant that the products do not include water in such amount that it will adversely affect the stability required by the remineralization products of this invention, i.e., the components of the products of this invention do not contain significant quantities of free water. However, they may contain salts with water of hydration. Preferably, the chewing gum products of this invention include either no water or only traces of water.

In the present invention, the demineralized subsurface of a tooth is effectively remineralized and/or tubules in exposed dentin are effectively mineralized when an effective amount of a product within the scope of this invention is used. As used herein with respect to the amount of the product used in the methods of this invention, the term "effective amount" refers to an amount which, when used in accordance with this invention, will bring about the remineralizing of teeth having carious lesions formed in the subsurfaces thereof, or the mineralizing of tubules in exposed dentin.

In the products of this invention, the calcium and phosphate salts are each used in a concentration ranging from about 0.05 to 15.0% by weight or the limit of solubility of the salt, preferably from about 0.10% to about 10.0% by weight. In one preferred embodiment of the chewing gum product of this invention, the calcium and phosphate salts are each used in a concentration ranging from 0.01 to 15.0% by weight or the limit of solubility of the salt, preferably from 0.10% to 10.0% by weight. In the products of this invention, excess calcium or phosphate salt can be present, if desired. The concentration of the calcium salt(s) containing the desired remineralizing cations is essentially the same as the concentration of the phosphate salt(s) containing the desired remineralizing anions.

In the products of this invention, the concentration of the soluble, non-toxic divalent metal salts (other than calcium) is greater than 0.001%, preferably between 0.001% to 2.0%, more preferably from 0.01% to 1.0%. In a preferred embodiment of the chewing product of this invention, the divalent metal salt(s) is present at a concentration level of greater than 0.0002% by weight, preferably from 0.0002% to 1.0% by weight, most preferably from 0.01% to 1.0% by weight.

Although many precipitates are within the broad scope of this invention, by depositing a precipitate less soluble than the original enamel, the remineralized subsurface can be made to be more resistant to demineralization than was the original enamel. If larger crystals are formed than were originally present, the subsurface will be somewhat more resistant to demineralization because of the lower surface area for attack. Providing a controlled rate of remineralization using this invention may promote formation of such larger crystals. If a fluoride ion utilized, the remineralized enamel is more resistant to demineralization than was the original enamel. In the products of the present invention, the concentration of fluoride salt preferably ranges from 0.01% to 5.0% by weight, more preferably from 0.02% to 2.0% by weight. However, in preferred embodiments of the chewing gum product of the present invention, the concentration of fluoride salt(s) preferably ranges from 0.0001% to 0.5% by weight, more preferably from 0.0002% to 0.01% by weight. In chewing gum products, high levels of fluoride is undesirable because of the potential for dental fluorosis and other toxic effects.

In order to effect remineralization of the dental enamel, an effective amount of the desired cations and anions must be employed in the oral cavity. The amount of solution generated in the mouth must contain at least 100 ppm of desired cations and 100 ppm of desired anions and preferably contains more than 1,000 ppm of desired cations and 1,000 ppm of desired anions. The solution must contain at least 10 ppm of divalent metal ions other than calcium ions and, preferably contains more than 100 ppm thereof. Furthermore, in the products of this invention other than the chewing gum or edible products, it is preferred to provide a level of fluoride ions of from 20 ppm to 5,000 ppm in the oral cavity from the product. In chewing gum or edible products, the level of fluoride ions generated in the oral cavity preferably ranges from 0 ppm to 5000 ppm, more preferably from 0 ppm to 50 ppm.

While the length of time of contact between the dissolved calcium and phosphate salts and the tooth's surface is not critical, it is necessary for the length of time to be great enough to allow diffusion of the ions through the tooth's surface to the demineralized subsurface. With products other than chewing gum products, the length of time is preferably at least ten seconds, more preferably greater than thirty seconds and even longer if possible. With chewing gum products, the length of time is preferably greater than one minute, more preferably greater than 15 minutes and even longer if possible. The desired extended time for such diffusion is a benefit accruing from the use of the divalent metal salts in this invention.

Upon preparation with water or upon use in the oral cavity with saliva (e.g., with respect to chewing gums, upon mastication thereof), any solution generated from a product within the scope of this invention should have a pH of from 4.5 to 7.0, preferably between 5.0 and 7.0, and more preferably between 5.0 and 5.75, both before and after the precipitation reaction, and be otherwise compatible in the oral environment. The ions must not combine prematurely in the solution to form a precipitate, but must be able to diffuse through the surface of the tooth to a demineralized subsurface area and be able to form an insoluble salt with ions of the other solution.

In two-part products within the scope of this invention, the pH of each part has a pH of at least about 3. However, the aqueous mixed solution formed from the two parts and which is used in the oral cavity must have a pH in the range of from 4.5 to 7.0, preferably from 5.0 to 7.0, more preferably from 5.0 to 5.75. The pH values of each of the two parts in the two-part products can be adjusted so long as the above pH parameters are not exceeded. The pH values of the parts in the two-part products of this invention as well as the pH values of the one-part products of this invention can be adjusted by methods known in the art. For example, the pH may be lowered by adding any acid which is safe for use in the oral cavity, by adding a base or by adding a buffering agent. Non-limiting examples of suitable acids include acetic acid, phosphoric acid, hydrochloric acid, citric acid and malic acid. Non-limiting examples of suitable bases include sodium hydroxide. Examples of suitable buffering agents include sodium citrate benzoate, carbonate, or bicarbonate, disodium hydrogen phosphate, or sodium dihydrogen phosphate. Preferably, the remineralizing salts employed can be selected to obtain the desired pH. Usually a combination of monobasic, dibasic and/or tribasic alkali metal phosphate salt is selected to provide the target pH.

The solutions and insoluble precipitates produced from the products of this invention must have acceptable levels of toxicity (i.e., the particular ions, in the amounts used in the remineralization process, must be non-toxic).

As mentioned previously, one preferred embodiment of the invention is directed to a remineralizing/mineralizing product in the form of a stable, one-part dry-mix. Such dry-mix contains from 1.0% to 80.0% of the at least one calcium salt, from 1.0% to 80.0% of the at least one phosphate salt, from 0.1% to 20.0% of the at least one water-soluble divalent metal salt, wherein the metal is other than calcium, from 0.1% to 20.0% of flavor (preferably provided as a spraydried powder), from 0.1% to 30.0% of sweetener, from 0 to 10.0% of a fluoride salt, and from 0 to 5.0% of a surfactant.

As also mentioned hereinabove, chewing gum products of this invention should contain 0.005% to 20.0%, preferably 0.1% to 7.0%, of the aforementioned dry-mix. It is preferable to provide sufficient calcium and phosphate salt in the gum to ensure that the concentration of each exceed 100 ppm and preferably 1000 ppm for an extended period.

In two-part products of the present invention, the remineralizing cationic part of a dentifrice composition contains 0.05% to 15.0%, preferably 0.10% to 10.0%, of at least one soluble calcium salt yielding calcium ions and greater than 0.001%, preferably from 0.001% to 2.0%, most preferably from 0.01% to 1.0%, of at least one water-soluble divalent metal salt selected from the group consisting of magnesium, strontium, tin and zinc, with magnesium being preferred. The remineralizing anionic part of the two-part product contains from 0.05% to 15.0%, preferably 0.10% to 10.0%, of dissolved phosphate salt yielding phosphate ions and from 0.01% to 5.0%, preferably from 0.02% to 2.0%, of a soluble fluoride salt yielding fluoride ions.

As mentioned previously herein, a particularly preferred embodiment of this invention is directed to a remineralizing/mineralizing product in the form of a chewing gum. The cationic phase of such gum contains 0.05% to 15.0%, preferably 0.10% to 10.0%, of the at least one soluble calcium salt yielding calcium ions and greater than 0.0002%, preferably from 0.0002% to 1.0%, most preferably from 0.01% to 1.0%, of the at least one water-soluble divalent metal salt selected from the group consisting of magnesium, strontium, tin and zinc, with magnesium being preferred. The remineralizing anionic phase of the gum contains from 0.01% to 15.0%, preferably 0.10% to 10.0%, of dissolved phosphate salt yielding phosphate ions. From 0.0001% to 0.5%, preferably from 0.0002% to 0.01%, of the soluble fluoride salt yielding fluoride ions may be employed. Here too, suitable pH adjusting compounds are employed so that the resulting pH in use is between 4.5 and 7.0, preferably between 5.0 and 7.0, and more preferably between 5.0 and 5.75.

The resulting precipitate formed from the products and methods of this invention is a calcium phosphate or hydroxyapatite, the natural constituent of tooth enamel, with incorporated fluoride ions. Not only do the improved processes of the present invention result in remineralized enamel, but the remineralized enamel may be more resistant to subsequent demineralization than was the original enamel.

As the calcium compound it is, in principle, possible to employ, in the preparations of the invention, any water-soluble toxicologically harmless calcium compound. A compound is considered to be water-soluble when at least 0.25 gram thereof dissolves in 100 ml of H₂O at 20°C.

Suitable water-soluble calcium compounds are, for example, calcium chloride, calcium bromide, calcium nitrate, calcium acetate, calcium gluconate, calcium benzoate, calcium glycerophosphate, calcium formate, calcium fumarate, calcium lactate, calcium butyrate and calcium isobutyrate, calcium malate, calcium maleate, calcium propionate, or mixtures of water-soluble calcium compounds. Calcium lactate is preferred. In the compositions of the invention for the remineralization of human dental enamel, at least 100 ppm and preferably at least 1000 ppm of calcium ions should be present; the upper limit is 35,000 ppm of calcium ions.

Suitable water-soluble inorganic phosphates within the scope of the present invention are, for example, alkali salts and ammonium salts of orthophosphoric acid, such as potassium, sodium or ammonium orthophosphate, monopotassium phosphate, dipotassium phosphate, tripotassium phosphate, monosodium phosphate, disodium phosphate and trisodium phosphate. The concentration of the phosphate ions is at least 100 ppm, and preferably at least 1000 ppm to 40,000 ppm. Solubility in water is defined as in the case of the calcium compounds.

If desired, water-soluble salts yielding both calcium and phosphate ions, such as monobasic-calcium orthophosphate, may be employed.

As the stabilizing divalent metal compound it is also, in principle, possible to employ any water-soluble, non-toxic divalent metal compound which will stabilize the calcium and phosphate ions so that they do not rapidly or prematurely precipitate before diffusing into the teeth. In practice, however, it has been found that at least one member selected from the group consisting of magnesium, strontium, tin, and zinc, with magnesium being preferred, are the most effective in stabilizing the system.

Suitable magnesium compounds are, for example, magnesium acetate, magnesium ammonium sulfate, magnesium benzoate, magnesium bromide, magnesium borate, magnesium citrate, magnesium chloride, magnesium gluconate, magnesium glycerophosphate, magnesium hydroxide, magnesium iodide, magnesium oxide, magnesium propionate, magnesium D-lactate, magnesium DL-lactate, magnesium orthophosphate, magnesium phenolsulfonate, magnesium pyrophosphate, magnesium sulfate, magnesium nitrate, and magnesium tartrate. Preferred magnesium compounds are magnesium chloride, magnesium acetate and magnesium oxide.

Suitable strontium compounds are, for example, strontium acetate, strontium ammonium sulfate, strontium benzoate, strontium bromide, strontium borate, strontium caprylate, strontium carbonate, strontium citrate, strontium chloride, strontium gluconate, strontium glycerophosphate, strontium hydroxide, strontium iodide, strontium oxide, strontium propionate, strontium D-lactate, strontium DL-lactate, strontium pyrophosphate, strontium sulfate, strontium nitrate, and strontium tartrate. Preferred strontium compounds are strontium acetate, strontium chloride, strontium nitrate.

Suitable tin compounds are, for example, stannous acetate, stannous ammonium sulfate, stannous benzoate, stannous bromide, stannous borate, stannous carbonate, stannous citrate, stannous chloride, stannous gluconate, stannous glycerophosphate, stannous hydroxide, stannous iodide, stannous oxide, stannous propionate, stannous D-lactate, stannous DL-lactate, stannous orthophosphate, stannous pyrophosphate, stannous sulfate, stannous nitrate, and stannous tartrate. A preferred tin compound is stannous chloride.

Suitable zinc compounds are, for example, zinc acetate, zinc ammonium sulfate, zinc benzoate, zinc bromide, zinc borate, zinc citrate, zinc chloride, zinc gluconate, zinc glycerophosphate, zinc hydroxide, zinc iodide, zinc oxide, zinc propionate, zinc D-lactate, zinc DL-lactate, zinc pyrophosphate, zinc sulfate, zinc nitrate, and zinc tartrate. Preferred zinc compounds are zinc acetate, zinc chloride, zinc sulfate, and zinc nitrate.

The concentration of divalent metal ions is at least 10 ppm, and preferably at least 100 ppm, with 20,000 ppm or more being the upper limit. solubility in water is, again, as defined as in the case of calcium and phosphate compounds.

The compositions of the invention for the remineralization or prevention of demineralization of human teeth may also contain water-soluble fluoride compounds, the caries-prophylactic activity of which has for a long time been considered to be established, In two-part products, the fluoride salt(s) is preferably present in the phosphate-containing part so as to avoid the formation of sparingly soluble calcium fluoride. In the products of this invention other than the chewing gum products, the fluoride salt is preferably present in an amount of from 0.01% to 5.0% by weight, more preferably from 0.02% to 2.0% by weight. In chewing gum products, however, because of the potential for fluorosis or other toxic effects, the concentration of fluoride salt(s) should preferably not exceed 0.1% by weight.

Suitable fluoride compounds are the alkali fluorides such as sodium, potassium, lithium or ammonium fluoride, tin fluoride, indium fluoride, zirconium fluoride, copper fluoride, nickel fluoride, palladium fluoride, fluorozirconates such as sodium, potassium or ammonium fluorozirconate or tin fluorozirconate, fluorosilicates, fluoroborates, fluorostannites.

Organic fluorides, such as the known amine fluorides are also suitable for use in the compositions of the invention.

Water-soluble alkali metal monofluorophosphates such as sodium monofluorophosphate, lithium monofluorophosphate and potassium monofluorophosphate, preferably, sodium monofluorophosphate may be employed In addition other water-soluble monofluorophosphate salts may be employed Including ammonium monofluorophosphate aluminum monofluorophosphate, and the like. If monofluorophosphate salts are used as the fluoride source in two-part products, such salts could be present in the first part along with the calcium cations without departing from the present invention. However, this is less desirable due to the potential loss of fluoride.

In one-part products of this invention, e.g, one-part chewing gum products, it has been found that even with the divalent metal salt used in the present invention, some reaction between the calcium and phosphate ions still, in fact, may take place and cause some formation of insoluble calcium phosphate, etc. during storage. The present invention optionally overcomes this problem by including suitable stabilizing means or the like, either in addition to or instead of said divalent metal salts, which prevent reaction of the calcium ions with the phosphate ions and also with the fluoride ions if they are present.

Any orally acceptable material that stabilizes one or more of the calcium, phosphate and/or fluoride salts and prevents reaction of the salts with each other during storage of the product (e.g., the chewing gum composition) in a closed container or package can be employed in the present invention. Examples of suitable stabilizing agents or stabilizing means include desiccating agents, coating or encapsulating materials, and mixtures of such stabilizing agents.

Examples of suitable desiccating agents include magnesium sulfate, sodium sulfate, calcium sulfate, calcium chloride, and colloidal silica, e.g., colloidal silica particles sintered together in chain-like formations having surface areas of from about 50 to about 400 square meters per gram such as materials sold under the trademark Cab-O-Sil by Cabot Corporation. It is believed that such materials stabilize the products of this invention by, for example, absorbing any existing water either present in or contacted with the composition so as to prevent reaction of the calcium, phosphate and/or fluoride salts.

When used, the stabilizing material is included in the products of the invention in an amount effective so as to inhibit reaction between the calcium, phosphate and, if present, fluoride salts in the product during storage in a closed container, but so as to allow release of sufficient calcium, phosphate and, if present, fluoride ions when the product is contacted with saliva, e.g., during mastication of the chewing gum. Typically, the stabilizing material is included in the products of this invention in an amount of up to about 7.5%, preferably from 0.1% to 5.0%.

One embodiment of a stable, single-part, non-aqueous chewing gum product of this invention comprises: (i) from 0.01% to 15.0%, preferably 0.10% to 10.0% of the water-soluble calcium salt; (ii) from 0.01% to 15.0%, preferably 0.10% to 10.0%, of the water-soluble phosphate salt;
(iii) from 0 to 7.5% of an orally acceptable desiccating agent;
(iv) from 10.0% to 95.0% of a gum base, and
(v) wherein when the salts are contacted with saliva the pH is between 4.5 and 7.0, preferably between 5.0 and 7.0, more preferably between 5.0 and 5.75.

Another method for inhibiting premature reaction of the calcium, phosphate and/or fluoride salts in one-part products (e.g., chewing gum products) of this invention is to provide a coating on or encapsulation thereof, e.g., with an oleophilic or, preferably, a polymeric material, which prevents reaction between the active materials. The presence of the coating on the various salts in the products of this invention prevents reaction of the active material by other substances, for example, by traces of water in or absorbed into the system. Preferably, the coating is edible coating. Suitable encapsulating or coating materials include oleophilic and other materials such as conventional edible gums, polymers which exhibit proportion ranging from hydrophilic to hydrophobic (water-insoluble), resins, waxes and mineral oils. The coating is preferably rinsable from the mouth.

In accordance with the invention, a polymer employed for coating the water-soluble calcium and/or phosphate salt particles and the like of the invention is selected from hydrophilic organic polymers and hydrophobic (water-insoluble) organic polymers and mixtures thereof.

A hydrophilic polymer employed for coating the remineralizing salt particles is selected from water-soluble and water-dispersible organic polymers. A mixture of polymers can be employed, and a content of between 5.0% and 95.0% of a water-insoluble polymer, based on the coating weight, can be included with a hydrophilic polymer.

The term "hydrophilic" as employed herein refers to an organic polymer which has a water-solubility of at least about one gram per 100 grams of water at 25°C. The term "hydrophobic" or "water-insoluble" as employed herein refers to an organic polymer which has a water solubility of less than about one gram per 100 grams of water at 25°C.

Suitable hydrophilic polymers for coating the salt particles in this invention include, e.g., gum arabic, gum karaya, gum tragacanth, guar gum, locust bean gum, xanthan gum, carrageenan, alginate salt, casein, dextran, pectin, agar, sorbitol, 2-hydroxyethyl starch, 2-aminoethyl starch, maltodextrin, amylodextrin, 2-hydroxyethyl cellulose, methyl cellulose, carboxymethyl cellulose salt, cellulose sulfate salt, polyvinylpyrrolidone, polyethylene glycol, polypropylene glycol, polyethylene oxide, polyvinyl alcohol/acetate, and the like. Polyvinyl acetate is illustrative of a water-insoluble polymer which can be included as an additional coating component to moderate the hydrophilicity of a hydrophilic polymer coating.

Suitable water-insoluble polymers, alone or in combination with one or more other components, for coating remineralizing salt particles include polyvinyl acetate, polyacrylamide, polyvinyl chloride, polystyrene, polyethylene, polyurethane, and the like.

The application of the polymer coating to the blend of calcium, phosphate and other salt particles of the invention process is accomplished by conventional means such as pan coating, fluidized coating, centrifugal fluidized coating, and the like. The coating polymer usually is dissolved in a suitable solvent such as water, methanol, ethanol, acetone, tetrahydrofuran, ethyl acetate, dimethylformamide and the like, as appropriate for a selected polymer species. A coating polymer also can be applied in the form of an emulsion or suspension. After the coating medium is applied to the particles, the solvent medium is removed by evaporation, thereby forming a continuous film coating which encapsulates the discrete crystallite particles.

The coating thickness on the surface of the salt typically will vary in the range between about 0.1-20 microns. The coating can consist of a single layer or multiple layers. The polymeric coating can constitute between about 5.0% to 50.0% of the total dry weight of the coated particles.

For purposes of release of the core matrix remineralizing calcium phosphate and, if desired, fluoride salts in the encapsulated particles when introduced into the aqueous environment, a surface coating of water-insoluble polymer of the oral cavity may have a content between about 5.0% to 30.0% by weight of a particulate water-extractable organic or inorganic filler, such as sodium monosaccharide or disaccharide, sorbitol powder, mannitol, and the like.

The rate of release of remineralizing salt core matrix content of the encapsulated particles under aqueous conditions can be controlled by the quantity and type of polymer coating on the particle surface.

Low molecular weight hydrophilic polymers will release the particle core matrix content at a relatively fast rate in the presence of moisture. High molecular weight polymers which are less hydrophilic will release at a relatively slow rate. Additional rate control is obtained by using mixtures of polymer components of varied hydrophilicity.

Polyethylene glycol (M.W. of 4000) or polyvinyl alcohol will release the particle core matrix at a relatively fast rate. Polyethylene oxide (M.W. of 4,000,000) or partially hydrolyzed polyvinyl acetate will release at a relatively slow rate. Polyvinylpyrrolidone will release the particle core matrix content at an immediate rate, when the encapsulated particles incorporated into the solid product are applied to the teeth.

A present invention encapsulated remineralizing salt particle composition exhibits a unique combination of properties because of the novel physical form of the free-flowing, but substantially inert, polymer-coated particles when utilized as an ingredient in chewing gums.

Examples of suitable oleophilic coatings or encapsulating materials include paraffin, mineral oil, edible oils such as peanut oil, coconut oil, palm oil, or safflower oil, oleophilic organic esters such as isopropyl siloxane myristate or isopropyl palmitate, edible polysiloxanes; and the like.

Encapsulating salts with a mixture of paraffin and waxes is also suitable.

By employing mineral oil as an oleophilic coating material for the calcium, phosphate and/or fluoride salts in the products of the invention, one other advantageous characteristic is provided. Specifically, oral bacteria is known to be adversely affected by oleophilic materials. Thus, the mineral oil used in the products of the invention will help in removing undesired bacteria during the course of treatment.

The coating should be of a thickness and composition so that it either readily dissolves, disperses, or emulsifies in water, e.g., in the mouth during chewing, or disintegrates during such action to release the active materials, i.e., one or more salts.

If the oleophilic material used for the coating is water insoluble, such as mineral oil, the coating phase can be pre-emulsified with a non-ionic, non-aqueous surfactant such as a hydrophilic ethoxylated sorbitan monooleate, e.g., the material sold under the trademark Tween. In this manner, when the product is placed in water, the mineral oil or other oleophilic coating on the particles is emulsified more readily than without the emulsification agent being present. Other similar surfactants can be employed such as sodium lauryl sulfate and other non-ionic surfactants.

Another embodiment of a stable single-part non-aqueous chewing gum within the scope of this invention comprises:
(i) from 0.01% to 15.0%, preferably 0.10% to 10.0%, water-soluble calcium salt;
(ii) from 0.01% to 15.0%, preferably 0.10% to 10.0% water-soluble phosphate salt, optionally from 0.0001% to 0.5% and preferably from 0.0002% to 0.01% water-soluble fluoride salt;
(iii) wherein there is an encapsulating coating on at least one of the water-soluble salts that either readily dissolves, disperses or emulsifies in saliva;
(iv) from 10.0% to 95.0% of a gum base, and
(v) wherein when the salts are contacted with saliva the pH is between 4.5 and 7.0, preferably between 5.0 and 7.0, more preferably between 5.0 and 5.75.

The products of this invention can be prepared, e.g., as single solutions, as two separate solutions, as a single solid powdered, gel or paste form, as a solid powder, gel or paste comprised of two separate components, or as a one-part or two-part gum.

In accordance with this invention, a single solution comprised of the ingredients set forth below can be prepared as follows. At least one water-soluble calcium salt in a concentration of from 0.05% to 15.0% is dissolved. At least one water-soluble divalent metal salt, other than a calcium salt, is added in a concentration greater than 0.001% and preferably from 0.001% to 2.0%. A second solution containing at least one water-soluble phosphate salt at a concentration of from 0.05% to 15.0% is also prepared. If desired, at least one water-soluble fluoride releasing compound may be added to the phosphate containing solution at a concentration of from 0.01% to 5.0%. The pH of each solution is adjusted so that upon mixing the final total solution pH will be between 4.5 and 7.0, preferably between 5.0 and 7.0, more preferably between 5.0 and 5.75. The two solutions are then mixed to produce a stable supersaturated or nearly supersaturated solution of calcium phosphate. In this preferred embodiment of the invention, it is preferable to use sodium monofluorophosphate as the fluoride source. This keeps the fluoride in solution even in the presence of high concentrations of calcium and allows the supersaturated calcium phosphate solutions to remain stable for longer periods.

When the total concentration of calcium ions and phosphate ions is such that the mixing results in a metastable solution, i.e., one that eventually precipitates calcium phosphate on standing, the two solutions are kept separately until it is desired to use the admixed product and obtain the advantage of the stabilizing divalent metal ions of the present invention.

A particularly preferred embodiment of this invention is directed to a two-part product comprising: (i) a first discrete part containing from 0.05% to 15.0%, preferably 0.10% to 10%, of the at least one water-soluble calcium salt together with from 0.001% to 2.0%, preferably 0.01% to 1.0%, of the at least one water-soluble, non-toxic divalent metal salt other than a calcium salt; (ii) a second discrete part comprising from 0.05% to 15.0%, preferably 0.10% to 10%, of the at least one water-soluble phosphate salt, and if desired, from 0.01% to 5.0% and preferably from 0.02% to 2.0% of the at least one fluoride releasing agent, (iii) a dispensing container comprising at least two discrete compartments each with an outlet end, the first compartment storing the first part which includes soluble calcium salt together with soluble divalent metal salt and the second compartment storing the second part which includes soluble phosphate salt and, if desired, together with the fluoride source, (iv) a closure mechanism for closing the compartments; and (v) wherein when the two parts are mixed the pH is between 4.5 and 7.0, preferably between 5.0 and 7.0, more preferably between 5.0 and 5.75.

A plurality of packaging methods may be employed in order to separately contain or store the two parts and provide effective dispensing thereof into the oral cavity.

Thus, the two parts of a toothpaste, gel, cream, or the like, may be simultaneously dispensed from separate collapsible tubes preferably made of plastic, a plastic and metal laminate. For convenience and in order to aid in dispensing substantially equal amounts of the parts, the tubes may be held together by banding or cementing, preferably along the corresponding ventral sides of the tubes.

In another embodiment, the two tubes may be constructed to have abutting, preferably flat, sidewall portions. The mouths of the tubes are usually close enough to allow sufficient quantities of the first and second parts to be simultaneously dispensed directly into the oral cavity or onto a substrate, e.g., a toothbrush, which delivers the two parts to the oral cavity, with the tubes being capped separately.

Alternatively, another packaging method comprises loading each part into separate compartments of the same collapsible composite tube, joined by a common orifice. Such composite tube has compartments separated by a divider which is firmly attached along substantially diametrically opposed portions of the sidewall and corresponding portions of the head structure of the tube. The divider may be glued or welded to the sidewall and head structure of the tube during manufacture of the latter. The divider is preferably provided with a protruding portion which extends into the mouth of the tube until its edge is substantially flush with the rim of the mouth. Thus, a divider forms with the sidewall two separate compartments of substantially the same volume for storage of the first and second parts, respectively.

In another alternative packaging method, the two tubes are concentric, wherein an inner tube lies within and parallel with an outer tube. The mouths of the tubes abut at the same point. Protrusions or the like are inserted between the inner and outer tubes so that the part contained in the outer tube can pass through an available space between the mouth of the outer tube and the mouth of the inner tube. The closures of this tube-within-a-tube, (which can screw on the outer tube or simply be held by pressure), may, but does not have to be, equipped with an interior protrusion to fit in the inner tube in order to prevent premature intermixing of the two parts at the mouth of the tube.

The tubes of all the above embodiments are usually filled from the bottom and are subsequently sealed together by conventional techniques.

Another alternative packaging arrangement comprises a pressurized container which is provided with two compartments and two spouts. The internal pressure of the compartments is maintained by a pressurized gas, i.e., nitrogen, at the bottom of each compartment. Operation of a mechanical actuator actuates valves which release the contents of the compartments through the spouts causing discharge of the two parts onto a brush.

The mouthwash or rinse and similar liquid embodiments are maintained in a manner similar to the pastes or gels in that during storage, each of the parts are maintained separate from one another to prevent premature reaction. Upon dispensing, the parts mix and react in the oral cavity to effect remineralization of dental enamel. The liquid parts can therefore be stored each in separate compartments of a dual-compartment dispenser. The dispenser usually includes a closure system comprising for example, an inclined crown portion, at least two pouring spouts extending upwardly from an upper surface of the crown portion and a cover for securement to the crown portion. The cover is provided with closure means, for example, depending plugs, to close the closure. Each pouring spout is preferably provided with a vent opening in addition to product orifices in the spouts. The orifices can be positioned close together on the crown, all of which assists in achieving control over pouring. Transparent containers have proven to be the most satisfactory. Transparency aids a person's ability to accurately and controllably dispense relatively equal volumes from a dual-compartment dispenser. Transparent walled containers also serve as a window function for gauging the amounts of liquid remaining in the dispenser. The walls of the containers can be scribed or otherwise calibrated to assist in dispensing the correct remineralizing amount of product.

Suitable toothpastes and gels can be made by employing, in addition to the remineralizing agents of the invention, from 0.5% to 65%, preferably from 5% to 40%, by weight of an abrasive; from 0.2% to 5% by weight of a sudsing agent; from 0.1% to 5% by weight of a binding agent; from 0% to 50% by weight of a humectant; and the balance, water and minors. From 1.0% to 10.0% of an inorganic thickener such as hydrated silica may be added.

In the case of two-part aqueous products, e.g., toothpastes, gels, mouthwashes and the like, the cationic and anionic parts of such product each has a pH of more than about 3. The mixture of the two portions which is placed in the mouth, however, must have a pH between 4.5 and 7.0, preferably between 5.0 and 7.0, more preferably between 5.0 and 5.75. The pH's of the cationic portion and the anionic portion can be adjusted so long as the above pH parameters are not exceeded.

Suitable abrasives include silica xerogels. Other conventional toothpaste abrasives can be used in the compositions of this invention, and include beta-phase calcium pyrophosphate, dicalcium phosphate dihydrate, anhydrous calcium phosphate, calcium carbonate, zirconium silicate, and thermosetting polymerized resins. Silica aerogels and the insoluble metaphosphates such as insoluble sodium metaphosphate can be used. Mixtures of abrasives can be also be used. Silica xerogel abrasives are preferred.

Suitable sudsing agents are those which are reasonably stable and form suds throughout the period of application. Preferably, non-soap anionic or nonionic organic synthetic detergents are employed. Examples of such agents are water-soluble salts of alkyl sulfate having from 10 to 18 carbon atoms in the alkyl radical, such as sodium lauryl sulfate, water-soluble salts of sulfonated monoglycerides of fatty acids having from 10 to 18 carbon atoms, such as sodium monoglyceride sulfonate, salts of C₁₀-C₁₈ fatty acid amides of taurine, such as sodium N-methyl taurate, salts of C₁₀-C₁₈ fatty acid esters of isethionic acid, and substantially saturated aliphatic acyl amides of saturated monoaminocarboxylic acids having 2 to 6 carbon atoms, and in which the acyl radical contains 12 to 16 carbon atoms, such as sodium-N-lauryl sarcoside. Mixtures of two or more sudsing agents can be used.

A binding material can be added to thicken and provide a desirable consistency for the present compositions. Suitable thickening agents are water-soluble salts of cellulose ethers, such as sodium carboxymethyl cellulose, hydroxypropyl cellulose, and hydroxyethyl cellulose. Natural gums such as gum karaya, gum arabic, carrageenan and gum tragacanth, can also be used. Colloidal magnesium aluminum silicate, silica aerogels, silica xerogels, fumed silica, or other finely divided silica can be used as part of the thickening agent for further improved texture. A preferred thickening agent is xanthan gum.

It is also desirable to include some humectant material in a toothpaste or gel to keep tt from hardening. Suitable humectants include glycerine, sorbitol, polyethylene glycol, propylene glycol, and other edible polyhydric alcohols as well as mixtures thereof.

Toothpaste or gel compositions within the scope of this invention may also contain flavoring agents, such as, e.g., oil of wintergreen, oil of peppermint, oil of spearmint, oil of sassafras, and oil of clove; sweetening agents such as, e.g., saccharin, dextrose, levulose, sodium cyclamate, and aspartame; and mixtures of sugar with a sweetener, e.g., sucralose.

It is also possible to manufacture the dentifrice product in the form of a transparent or translucent gel. This is accomplished by matching the refractive index of the water-humectant system with the abrasives and inorganic thickeners, if used.

Professional gels can be formulated similar to dentifrices but with higher fluoride contents. Since these products are not designed for cleaning but only as a fluoride application, abrasives and other cleaning agents need not be included in the formulation.

The remineralizing systems herein can also be provided in the form of a mouthwash product. Both the cationic and anionic parts of mouthwashes can be made in accordance with the following. Mouthwashes generally comprise an aqueous solution of ethyl alcohol and flavoring materials. The alcohol provides an antibacterial effect, solubilizes the flavoring materials and provides a pleasant mouth feeling. Alcohol-free mouthwashes are now, however, gaining in popularity. Optionally, mouthwashes also contain additional antibacterial agents and humectants, such as glycerine and sorbitol, which give a moist feeling to the mouth.

An alcohol-free mouthwash within the scope of this invention is preferably provided in the form of a dry powder or tablets which are added to water immediately before use. Such a product would contain from 10% to 95% by weight of the water-soluble salts and the balance would be flavoring agents, sweeteners, and, optionally, antibacterial agents.

In addition to the remineralizing agents of the invention, typical mouthwashes contain 0 to 30%, preferably 0 to 20%, ethyl alcohol; 30% to 90% water; 0 to 20% glycerine or other humectant; 0 to 0.1% of an antibacterial agent; 0 to 0.2% of a soluble fluoride source; 0.01% to 0.5% of a sweetening agent; 0.01% to 2.0% of a flavoring agent; and from 0.1% to 1% of an emulsifier-surfactant.

Examples of suitable flavoring agents include heliotropyl nitrile, wintergreen (methyl salicylate), oil of peppermint, oil of assia, oil of anise, oil of cinnamon, and mixtures thereof. Suitable sweetening agents include, e.g., saccharin, glycerine, sorbitol, levulose, and 6-(trifluoromethyl)-tryptophane and aspartyl phenylalanine methyl ester.

As stated previously herein, remineralizing products within the scope of this invention may also be in the form of chewing gum. The chewing gum may be any of a variety of different chewing gums, bubble gums, dragees, and the like, including low or high moisture, sugar or sugarless, wax-containing or wax-free, low calorie (via high base or low calorie bulking agents), and/or may contain other dental health agents.

Chewing gum products within the scope of this invention may be prepared as follows. A gum base is melted at a temperature, e.g., of from about 85°C to about 90°C, cooled to a temperature of about 78°C, and then placed in a pre-warmed (60°C) standard mixing kettle equipped with sigma blades. The emulsifier is then added. Next, sorbitol and glycerin are added and the ingredients are mixed for an additional 3 to 6 minutes. The mixing kettle is cooled and mannitol and the remainder of the sorbitol and glycerin are then added and mixing is continued. At this time, the unflavored chewing gum has a temperature of from about 39°C to about 42°C. Flavor oil is then added and incorporated into the base and mixing is continued. Finally, the sweetener material is added and mixed for an additional 1 to 10 minutes. The remineralization system is added as the last ingredient. The final gum temperature is from about 39°C to about 43°C. The chewing gum composition is then discharged from the kettle, rolled, scored and formed into chewing gum pieces.

Chewing gum generally consists of a water insoluble gum base, a water soluble portion, and flavors. The water soluble portion dissipates with a portion of the flavor over a period of time during chewing. The gum base portion is retained in the mouth throughout the chew.

The insoluble gum base generally comprises elastomers, resins, fats and oils, softeners, and inorganic fillers. The gum base may or may not include wax. The insoluble gum base can constitute about 5% to 95%, by weight, of the chewing gum, more commonly, the gum base comprises about 10% to 50% of the gum, and in some preferred embodiments, about 20% to 35%, by weight, of the chewing gum.

In an embodiment, the chewing gum base of the present invention contains about 20% to 60% synthetic elastomer, 0 to about 30% natural elastomer, about 5% to 55% elastomer plasticizer, about 4% to 35% filler, about 5% to 35% softener, and optional minor amounts (about one percent or less) of miscellaneous ingredients such as colorants, antioxidants, etc.

Synthetic elastomers may include, but are not limited to, polyisobutylene with GPC weight average molecular weight of about 10,000 to 95,000, isobutylene-isoprene copolymer (butyl elastomer) styrene-butadiene copolymers having styrene-butadiene ratios of about 1 to 3 up to 3 to 1, polyvinyl acetate having GPC weight average molecular weight of about 2,000 to about 90,000, polyisoprene, polyethylene, vinyl acetate-vinyl laurate copolymer having vinyl laurate content of abotu 5% to 50% oaf the copolymer, and combinations thereof.

Preferred ranges are, for polyisobutylene, 50,000 to 80,000 GPC weight average molecular weight, for styrene-butadiene, 1 to 1 up to 1 to 3 bound styrene-butadiene, for polyvinyl acetate, 10,000 to 65,000 GPC weight average molecular weight with the higher molecular weight polyvinyl acetates typically used in bubble gum base, and for vinyl acetate-vinyl laurate, vinyl laurate content of about 10% to 45%.

Natural elastomers may include natural rubber such as smoked or liquid latex and guayule as well as natural gums such as jelutong, lechi caspi, perillo, sorva, massaranduba balata, massaranduba chocolate, nispero, rosindinha, chicle, gutta hang kang, and combinations thereof. The preferred synthetic elastomer and natural elastomer concentrations vary depending on whether the chewing gum in which the base is used is abhesive or conventional, bubble gum or regular gum, as discussed below. Preferred natural elastomers include jelutong, chicle, sorva and massaranduba balata.

Elastomer plasticizers may include, but are not limited to, natural rosin esters such as glycerol esters of partially hydrogenated rosin, glycerol esters polymerized rosin, glycerol esters of partially dimerized rosin, glycerol esters of rosin, pentaerythritol esters of partially hydrogenated rosin, methyl and partially hydrogenated methyl esters of rosin, pentaerythritol esters of rosin; synthetics such as terpene resins derived from alpha-pinene, beta-pinene, and/or d-limonene; and any suitable combinations of the foregoing. The preferred elastomer plasticizers will also vary depending on the specific application, and on the type of elastomer which is used.

Fillers/texturizers may include magnesium and calcium carbonate, ground limestone, silicate types such as magnesium and aluminum silicate, clay, alumina, talc, titanium oxide, mono-, di- and tricalcium phosphate, cellulose polymers, such as wood, and combinations thereof.

Softeners/emulsifiers may include tallow, hydrogenated tallow, hydrogenated and partially hydrogenated vegetable oils, cocoa butter, glycerol monostearate, glycerol triacetate, lecithin, mono-, di- and triglycerides, acetylated monoglycerides, fatty acids (e.g., stearic, palmitic, oleic and linoleic acids), and combinations thereof.

Colorants and whiteners may include FD%C type dyes and lakes, fruit and vegetable extracts, titanium dioxide, and combinations thereof.

The base may or may not include wax.

In addition to a water insoluble gum base portion, a typical chewing gum composition includes a water soluble bulk portion and one or more flavoring agents. The water soluble portion can include bulk sweeteners, high intensity sweeteners, flavoring agents, softeners, emulsifiers, colors, acidulants, fillers, antioxidants, and other components that provide desired attributes.

Softeners are added to the chewing gum in order to optimize the chewability and mouth feel of the gum. The softeners, which are also known as plasticizers and plasticizing agents, generally constitute between approximately about 0.5% to 15% by weight of the chewing gum. The softeners may include glycerin, lecithin, and combinations thereof. Aqueous sweetener solutions such as those containing sorbitol, hydrogenated starch hydrolysates, corn syrup and combinations thereof, may also be used as softeners and binding agents in chewing gum.

Bulk sweeteners include both sugar and sugarless components. Bulk sweeteners typically constitute 5 to about 95% by weight of the chewing gum, more typically, 20 to 80% by weight, and more commonly, 30 to 60% by weight of the gum.

Sugar sweeteners generally include saccharide-containing components commonly known in the chewing gum art, including, but not limited to, sucrose, dextrose, maltose, dextrin, dried invert sugar, fructose, levulose, galactose, corn syrup solids, and the like, alone or in combination.

Sugarless sweeteners include, but are not limited to, sugar alcohols such as sorbitol, mannitol, xylitol, hydrogenated starch hydrolysates, maltitol, and the like, alone or in combination.

High intensity artificial sweeteners can also be used, alone or in combination with the above. Preferred sweeteners include, but are not limited to sucralose, aspartame, salts of acesulfame, alitame, saccharin and its salts, cyclamic acid and its salts, glycyrrhizin, dihydrochalccones, thaumatin, monellin, and the like, alone or in combination. In order to provide longer lasting sweetness and flavor perception, it may be desirable to encapsulate or otherwise control the release of at least a portion of the artificial sweetener. Such techniques as wet granulation, wax granulation, spray drying, spray chilling, fluid bed coating, coacervation, and fiber extension may be used to achieve the desired release characteristics.

Usage level of the artificial sweetener will vary greatly and will depend on such factors as potency of the sweetener, rate of release, desired sweetness of the product, level and type of flavor used and cost considerations. Thus, the active level of artificial sweetener may vary from about 0.02% to 8.0%. When carriers used for encapsulation are included, the usage level of the encapsulated sweetener will be proportionately higher.

Combinations of sugar and/or sugarless sweeteners may be used in chewing gum. Additionally, the softener may also provide additional sweetness such as with aqueous sugar or alditol solutions.

If a low calorie gum is desired, a low caloric bulking agent can be used. Examples of low caloric bulking agents include: polydextrose; Raftilose, Raftilin; fructooligosaccharides (NutraFlora); Palatinose oligosaccharide; Guar Gum Hydrolysate (Sun Fiber); or indigestible dextrin (Fibersol). However, other low calorie bulking agents can be used.

A variety of flavoring agents can be used. The flavor can be used in amounts of about 0.1% to 15.0% of the gum, and preferably, about 0.2% to 5.0%. Flavoring agents may include essential oils, synthetic flavors or mixtures thereof including, but not limited to, oils derived from plants and fruits such as citrus oils, fruit essences, peppermint oil, spearmint oil, other mint oils, clove oil, oil of wintergreen, anise and the like. Artificial flavoring agents and components may also be used. Natural and artificial flavoring agents may be combined in any sensorially acceptable fashion.

The chewing gum may either be sugarless or sugar containing. However, in an embodiment of the present invention, the chewing gum is sugar containing. This also overcomes some of the sensory quality problems of sugarless gums. Still further, such a chewing gum overcomes some of the other problems of sugarless gum. For example, some sugarless gums may be poorly tolerated by some chewers, who manifest gastrointestinal disturbances, because of the sugar alcohols used in sugarless gums.

Another component which is optionally used in the chewing gum embodiment of the present invention is a dental abrasive. Dental abrasives are particularly valuable in chewing gums due to the polishing action which occurs during mastication. The term dental abrasive includes all manner and form of toothpaste, chewing gums, and the like. Specifically, dicalcium diphosphate dihydrate is the preferred dental abrasive of the chewing gum embodiment of the present invention. This particular material also serves to function as an alkaline buffer as described above.

Further dental abrasives which may be utilized in the chewing gum embodiment of the present invention include calcium carbonate, sodium metaphosphate, aluminum hydroxide, magnesium carbonate, calcium sulphate, silicas including aerogels and xerogels, and tricalcium phosphate. The amount of the dental abrasive employed in the chewing gum embodiment of the present invention is generally within the range of from about 1.0% to 30.0%, preferably from about 1.5% to about 20.0% by weight.

Yet another optional ingredient in the chewing gum embodiment of the present invention is the use of glycerine. In the chewing gum aspect of the present invention, glycerine serves to soften and maintain the chewability of the chewing gum for prolonged periods. The glycerine also adds to the sweetness of the composition. The glycerine is ordinarily added at levels of from about 0.01% to 10.0%, preferably at from about 0.2% to 5.0% by weight of the composition.

The present invention includes as additional optional components water or a monohydric alcohol at from about 2.0% to 99.0%, preferably at from about 5.0% to 70.0%, and most preferably from about 10.0% to 50.0% by weight of the composition. It is of course recognized that it is particularly valuable to use mixtures of water and the monohydric alcohol generally within the weight ratio of from about 20 to 1 up to 1 to 20, preferably from about 10 to 1 up to 1 to 10.

The preferred monohydric alcohols are methanol, ethanol, or isopropanol although other monohydric alcohols generally including those having up to 18 carbon atoms may be utilized in the present invention. The preferred monohydric alcohol is ethanol. It should be recognized that where the product will be ingested that only ethanol should be used.

While Applicants do not wish the scope of the present invention to be limited by theory, it is believed that the calcium, phosphate, and, optionally, the fluoride ions diffuse through the tooth surface to the demineralized subsurface and precipitate in the demineralized subsurface where they remineralize the tooth structure. This is attainable because sufficient calcium, phosphate, and fluoride ions remain soluble for a period of time sufficient to permit their diffusion into the demineralized subsurface of the dental enamel. This is accomplished by the use of the divalent metal cations of this invention and in the instance of two-part systems combining the particular ions just prior to their application to the tooth in a solution having a pH of 4.5 to 7.0, preferably from 5.0 and 7.0, more preferably from about 5.0 to about 5.75 at which pH enough of the calcium, phosphate and fluoride ions remain soluble for the period of time required to remineralize the lesions of the dental enamel.

Chemically equivalent concentrations are not necessary as long as the molar ratio of calcium and phosphate ions in the mixture is from 0.01 to up to 100 to 1. It is preferred that the ratio is from 0.2 to 1 to 5 to 1, and it is most preferred that the ratio is from 1:1 to 1.67 to 1, the ratio of calcium to phosphate in the range of the various less soluble calcium phosphate salts.

While completely aqueous solutions are preferred in certain embodiments (i.e., the non-chewing gum embodiments) of the present invention, non-aqueous solvents may be employed in combination with water. For example, suitable nonaqueous solvents include ethyl alcohol, glycerine and propylene glycol. Solvent systems suitable for use in the present invention are those which are capable of dissolving the salts employed in the invention and which are safe for use in the mouth.

With regard to the period of time of exposure of the solutions to the teeth, it is necessary that the length of time be great enough to allow diffusion of the ions into the demineralized subsurface. At least about ten seconds are required for this diffusion. The solution is preferably applied to the teeth for from about 10 seconds to about 15 minutes. At least about one minute is required for the release of the salts from the chewing gum of this invention and for diffusion. The chewing gum is preferably masticated with the teeth for from about 5 minutes to about 15 minutes or more. The pH of the solution remains relatively constant after its introduction into the oral cavity. Under some conditions, calcium phosphate readily precipitates at this pH, but most surprisingly while some of the precipitation may occur immediately and some small amount even before application to the teeth, substantially greater amounts of calcium, phosphate and fluoride ions remain in solution to diffuse into the teeth and remineralize the demineralized dental enamel due to the presence of the divalent metal cations hereinbefore described. It is believed that the ability of the solutions to provide ions for remineralization is greatest upon their first introduction into the oral cavity, thereafter decreasing, but at a rate less than that encountered when not employing the instant divalent metal cations.

When using two-part systems, the time period between the mixing of the first and second solutions and the application of the mixture to the teeth preferably should not exceed 1 minute, and more preferably is less than 1 minute. With a toothpaste, gel, and the like, mixing of the remineralizing ingredients is achieved on the surface of the teeth while brushing. With a chewing gum and the like, mixing is achieved on the surface of the teeth while chewing. The essence of the present invention lies in the mixing of components and the quick and timely application of the resulting solution or in the mixing and dissolution of the components when the gum is chewed in the mouth to result in an aqueous solution, wherein the solution will precipitate calcium phosphate, calcium fluoride, and calcium fluoro-apatite in the subsurface enamel of the teeth. Before such precipitation occurs, the mixture comprising the solution must quickly be applied to the teeth. Surprisingly, the solution can have a pH of from 4.5 to 7.0, preferably from 5.0 to 7.0, more preferably 5.0 to 5.75, to achieve this result. As stated previously herein, at a pH below about 3, demineralization occurs rapidly. A pH below 2.5 is generally undesirable from a safety standpoint.

The following Examples illustrate the present invention. In the Examples and elsewhere herein, parts and percent are by weight unless otherwise stated.

### EXPERIMENTAL

### Examples 1-3

Artificial lesions, about 50 µ deep, were formed in one surface of bovine enamel chips using a demineralizing Carbopol® gel, which was used to treat the specimens for 72 hours. The surface hardness of the surface to be treated was then measured.

The regimen cycle consisted of a 30-minute demineralization in a standard demineralizing solution followed by a 5-minute treatment of the test products diluted one part product to two parts human saliva, followed by a 60-minute remineralization in human saliva. Overnight, which was every fifth cycle, the specimens were kept with a layer of saliva and stored in a cold room. The test ran for three days, for a total of 15 demineralization/treatment/remineralization cycles.

For the treatment cycle, the two parts of the remineralizing test agents of the example were separately diluted one part product to two parts saliva and mixed together immediately before immersion of the enamel specimens.

The two-part oral remineralizing treatment was prepared as set forth below. The pH of each composition after mixing the two parts was approximately 5.5.

| | Control A | |
|---|---|---|
| | Part A | Part B |
| Calcium nitrate | 3.00 | 0.00 |
| Magnesium chloride | 0.00 | 0.00 |
| Monopotassium phosphate | 0.00 | 2.00 |
| Dipotassium phosphate | 0.00 | 0.70 |
| Sodium fluoride | 0.00 | 0.50 |
| Sodium MFP | 0.00 | 0.00 |
| Glycerine | 24.00 | 22.85 |
| Water | 73.00 | 73.95 |

### Example 1

| | Part A | Part B |
|---|---|---|
| Calcium nitrate | 3.0 | 0.00 |
| Magnesium chloride | 0.4 | 0.00 |
| Monopotassium phosphate | 0.0 | 2.10 |
| Dipotassium phosphate | 0.0 | 0.60 |
| Sodium fluoride | 0.0 | 0.50 |
| Sodium MFP | 0.0 | 0.00 |
| Glycerine | 24.0 | 22.85 |
| Water | 72.6 | 73.95 |

### Example 2

| | Part A | Part B |
|---|---|---|
| Calcium nitrate | 3.0 | 0.00 |
| Magnesium chloride | 0.8 | 0.00 |
| Monopotassium phosphate | 0.0 | 2.10 |
| Dipotassium phosphate | 0.0 | 0.60 |
| Sodium fluoride | 0.0 | 0.50 |
| Sodium MFP | 0.0 | 0.00 |
| Glycerine | 24.0 | 22.85 |
| Water | 72.2 | 73.95 |

### Example 3

| | Part A | Part B |
|---|---|---|
| Calcium nitrate | 3.0 | 0.00 |
| Magnesium chloride | 0.8 | 0.00 |
| Monopotassium phosphate | 0.0 | 2.40 |
| Dipotassium phosphate | 0.2 | 0.20 |
| Sodium fluoride | 0.0 | 0.00 |
| Sodium MFP | 0.0 | 1.80 |
| Glycerine | 24.0 | 22.85 |
| Water | 72.2 | 72.75 |

| Hardness increase | |
|---|---|
| Crest | 16 |
| Control A | 20.0 |
| Example 1 | 62 |
| Example 2 | 49.0 |
| Example 3 | 21.0 |

The results show that Examples 1 and 2 containing sodium fluoride and magnesium chloride significantly outperform Control A and Crest which do not contain magnesium chloride. Example 3 containing sodium monofluorophosphate and magnesium chloride performed equal to Control A and better than Crest. This is surprising since sodium monofluorophosphate is generally less effective at promoting remineralization than sodium fluoride.

### Example 4

The following formulations were prepared.

### Example 4

| | Part A | Part B |
|---|---|---|
| Calcium nitrate | 3.0 | 0.0 |
| Magnesium chloride | 0.8 | 0.0 |
| Monopotassium phosphate | 0.0 | 0.6 |
| Dipotassium phosphate | 0.0 | 2.1 |
| Glycerine | 24.0 | 22.85 |
| Water | 72.2 | 74.45 |

| | Control B* | |
|---|---|---|
| | Part A | Part B |
| Calcium nitrate | 0.0 | 0.0 |
| Magnesium chloride | 0.0 | 0.0 |
| Monopotassium phosphate | 0.0 | 0.0 |
| Dipotassium phosphate | 0.0 | 0.0 |
| Glycerine | 50.0 | 50.0 |
| Water | 50.0 | 50.0 |

| | | |
|---|---|---|
| *The pH of the composition prepared in Control B was adjusted to 5.5. | | |

A similar treatment regimen was performed as for Examples 1-3 except that in Test 1 the treatment time was 15 minutes using the formulation of Example 4. In Test II and Control Test III, the first cycle of the day was a 5-minute treatment with Crest and cycles 2, 3, 4, and 5 were a 15-minute treatment with the formulation of Example 4 or Control B, respectively. The 15-minute treatment time was chosen to replicate what might happen if this formulation was released from a product where the treatment time might be extended to 15 minutes. The three tests were also compared with a standard Crest treatment in which Crest treatment was applied 5 times per day for 5 minutes. The test was run for 3 days for a total of 15 cycles.

### Test I

Example 4
5 cycles/day
Hardness Increase: 10.0

### Test II

Example 4
4 cycles/day
Crest
1 cycle/day
Hardness Increase: 13

### Control Test III

Control B
4 cycles/day
Crest
1 cycle/day
Hardness Increase: 6.0

### Crest

5 cycles/day
Hardness Increase: 16

The results show that treatments with the non-fluoride containing remineralizing formulation was effective in remineralizing teeth. Test I illustrates it was slightly less effective on a one to one treatment basis with Crest. However, Test II compared to Control Test III illustrates it was more effective than Crest on a one to one treatment basis than Crest. When used with the fluoride toothpaste, the remineralizing treatments had an additive remineralizing effect. This demonstrates the likely positive effects of a non-fluoride product, i.e., a lozenge or candy containing the remineralizing ingredients if repeated several times a day, e.g., after eating.

### Examples 5-7

Examples 5-7 illustrate various embodiments of remineralizing formulation of the invention. A two-part oral remineralizing mouthwash and a two-part remineralizing toothpaste and a one-part toothpaste are prepared as follows.

### Example 5

| | Mouthrinse | |
|---|---|---|
| | A | B |
| Water | 73.8 | 75.9 |
| Sorbitol | -- | -- |
| Glycerine | 20.0 | 20.0 |
| Silica abrasive | -- | -- |
| Silica thickener | -- | -- |
| CMC | -- | -- |
| Carbowax 8000 | -- | -- |
| Sodium lauryl sulfate | -- | -- |
| Calcium nitrate tetrahydrate | 4.5 | 0.0 |
| Calcium chloride anhydrous | -- | -- |
| Dipotassium phosphate | -- | 0.5 |
| Monopotassium phosphate | -- | 3.0 |
| Magnesium chloride hexahydrate | 1.2 | 0.0 |
| Magnesium oxide | -- | -- |
| Sodium monofluorophosphate | 0.0 | 0.0 |
| Sodium fluoride | 0.0 | 0.1 |
| Flavor | 0.4 | 0.4 |
| Saccharin | 0.1 | 0.1 |

### Example 6

| | Toothpaste | |
|---|---|---|
| | A | B |
| Water | 21.7 | 21.5 |
| Sorbitol | 40.0 | 40.0 |
| Glycerine | 10.0 | 10.0 |
| Silica abrasive | 15.0 | 15.0 |
| Silica thickener | 6.0 | 6.0 |
| CMC | 1.0 | 1.0 |
| Carbowax 8000 | -- | -- |
| Sodium lauryl sulfate | 1.5 | 1.5 |
| Calcium nitrate tetrahydrate | 3.0 | 0.0 |
| Calcium chloride anhydrous | -- | -- |
| Dipotassium phosphate | -- | 0.2 |
| Monopotassium phosphate | -- | 2.3 |
| Magnesium chloride hexahydrate | 0.8 | 0.0 |
| Magnesium oxide | -- | -- |
| Sodium monofluorophosphate | 0.0 | 1.5 |
| Sodium fluoride | 0.0 | 0.0 |
| Flavor | 0.8 | 0.8 |
| Saccharin | 0.2 | 0.2 |

### Example 7

| | Toothpaste |
|---|---|
| Water | 0.0 |
| Sorbitol | 0.0 |
| Glycerine | 69.66 |
| Silica abrasive | 12.0 |
| Silica thickener | 4.0 |
| CMC | 0.0 |
| Carbowax 8000 | 5.0 |
| Sodium lauryl sulfate | 1.5 |
| Calcium nitrate tetrahydrate | -- |
| Calcium chloride anhydrous | 3.0 |
| Dipotassium phosphate | 0.0 |
| Monopotassium phosphate | 3.0 |
| Magnesium chloride hexahydrate | -- |
| Magnesium oxide | 0.4 |
| Sodium monofluorophosphate | 0.0 |
| Sodium fluoride | 0.24 |
| Flavor | 0.9 |
| Saccharin | 0.3 |

### Examples 8-10

To further demonstrate the beneficial effects of magnesium, strontium, zinc and stannous ions in stabilizing supersaturated solutions of calcium phosphate, the following examples and controls were prepared.

### Example 8

| | Part A |
|---|---|
| Calcium nitrate | 1.44 |
| Zinc chloride | 0.015 |
| Stannous chloride | 0.00 |
| Strontium chloride | 0.00 |
| Water | 73.545 |

| | Part B |
|---|---|
| Monopotassium phosphate | 0.37 |
| Dipotassium phosphate | 0.14 |
| Water | 74.49 |

### Example 9

| | Part A |
|---|---|
| Calcium nitrate | 1.44 |
| Zinc chloride | 0.00 |
| Stannous chloride | 0.01 |
| Strontium chloride | 0.00 |
| Water | 73.55 |

| | Part B |
|---|---|
| Monopotassium phosphate | 0.42 |
| Dipotassium phosphate | 0.07 |
| Water | 74.51 |

### Example 10

| | Part A |
|---|---|
| Calcium nitrate | 1.44 |
| Zinc chloride | 0.00 |
| Stannous chloride | 0.00 |
| Strontium chloride | 0.30 |
| Water | 73.26 |

| | Part B |
|---|---|
| Monopotassium phosphate | 0.42 |
| Dipotassium phosphate | 0.07 |
| Water | 74.51 |

| | Control C |
|---|---|
| | Part A |
| Calcium nitrate | 1.44 |
| Zinc chloride | 0.00 |
| Stannous chloride | 0.00 |
| Strontium chloride | 0.00 |
| Water | 73.56 |

| | Part B |
|---|---|
| Monopotassium phosphate | 0.42 |
| Dipotassium phosphate | 0.07 |
| Water | 74.51 |

In Examples 8-10 and Control Example C, the two parts of each example were mixed and the time taken for the solutions to become cloudy was measured with the following results:

| After Mixing | | | | |
|---|---|---|---|---|
| | Ex. 8 | Ex. 9 | Ex. 10 | Control C |
| pH Start | 5.6 | 5.45 | 5.54 | 5.60 |
| Minutes before becoming cloudy | 10 | 10 | 9 | 6 |

The results show that the three examples of the invention remained stable for longer than the control product which did not contain a divalent metal salt. The addition of the divalent metal thus allows more time for penetration into the tooth before precipitation occurs.

### Examples 11 and 12 and Control Example D

The following monofluorophosphate-containing examples were prepared.

### Example 11

| | Part A |
|---|---|
| Calcium nitrate | 1.28 |
| Magnesium chloride | 0.10 |
| Stannous chloride | 0.00 |
| Water | 73.545 |

| | Part B |
|---|---|
| Monopotassium phosphate | 0.91 |
| Dipotassium phosphate | 0.12 |
| Sodium monofluorophosphate | 0.43 |
| Water | 74.49 |
| pH Start | 5.64 |
| Minutes before becoming cloudy | 7.5 |

### Example 12

| | Part A |
|---|---|
| Calcium nitrate | 2.01 |
| Magnesium chloride | 0.01 |
| Stannous chloride | 0.01 |
| Water | 73.55 |

| | Part B |
|---|---|
| Monopotassium phosphate | 1.02 |
| Dipotassium phosphate | 0.08 |
| Sodium monofluorophosphate | 0.43 |
| Water | 73.47 |
| pH Start | 5.54 |
| Minutes before becoming cloudy | 10 |

### Control Example D

| | Part A |
|---|---|
| Calcium nitrate | 1.28 |
| Magnesium chloride | 0.00 |
| Stannous chloride | 0.00 |
| Water | 73.72 |

| | Part B |
|---|---|
| Monopotassium phosphate | 0.91 |
| Dipotassium phosphate | 0.12 |
| Sodium monofluorophosphate | 0.43 |
| Water | 73.55 |
| pH Start | 5.69 |
| Minutes before becoming cloudy | 2.5 |

The magnesium chloride and stannous chloride stabilized the solutions from early precipitation of calcium phosphate even in the presence of MFP as shown by the longer times before the solutions became cloudy.

Examples 11 and 12 and Control Example D show how divalent metals of the invention successfully retard the precipitation of calcium phosphates.

### Examples 13-15

Examples of suitable mouthwashes and toothpastes are shown below in Examples 13-15.

### Example 13

| | Professional Gel | |
|---|---|---|
| | A | B |
| Water | 28.3 | 30.9 |
| Glycerine | 10.0 | 10.0 |
| Sorbitol | 40.0 | 40.0 |
| Silica thickener | 12.0 | 12.0 |
| Xantham gum | 0.9 | 0.9 |
| Calcium Nitrate | 7.5 | 0.0 |
| Stannous chloride | 0.1 | 0.0 |
| Dipotassium phosphate | 0.0 | 0.2 |
| Monopotassium phosphate | 0.0 | 4.2 |
| Sodium fluoride | 0.0 | 1.0 |
| Flavor | 0.5 | 0.5 |
| Saccharin | 0.3 | 0.3 |

### Example 14

| | Mouthwash | |
|---|---|---|
| | A | B |
| Water | 73.6 | 75.8 |
| Glycerine | 10.0 | 10.0 |
| Sorbitol | 10.0 | 10.0 |
| Calcium Nitrate | 5.5 | 0 |
| Zinc chloride | 0.1 | 0 |
| Dipotassium phosphate | 0 | 0.2 |
| Monopotassium phosphate | 0 | 3.2 |
| Flavor | 0.5 | 0.5 |
| Saccharin | 0.3 | 0.3 |

### Example 15

| | Toothpaste (One-Part) |
|---|---|
| Glycerine | 64.06 |
| Silica abrasive | 15.0 |
| Silica thickener | 5.0 |
| Sodium lauryl sulfate | 1.5 |
| Calcium Nitrate | 5.0 |
| Strontium chloride | 0.2 |
| Dipotassium phosphate | 0.3 |
| Monopotassium phosphate | 3.2 |
| Sodium MFP | 0.76 |
| Flavor | 0.7 |
| Saccharin | 0.4 |
| Carbowax 8000 | 2.0 |

### Examples 16 and 17

Examples 16 and 17 illustrate dry-mix formulations which are used when added to and mixed with water. Example 17 illustrates a dry-mix product for use as an effervescent mouthwash.

### Example 16

| | |
|---|---|
| Calcium gluconate | 65.0 |
| Magnesium chloride | 10.0 |
| Monosodium phosphate | 12.5 |
| Disodium phosphate | 2.5 |
| Stannous fluoride | 2.5 |
| Flavor | 5.0 |
| Saccharin | 2.5 |
| Usage concentration | 1.2 grams/ounce |

### Example 17

| | |
|---|---|
| Encapsulated* calcium nitrate (97%) | 36.0 |
| Monopotassium phosphate | 30.0 |
| Malic acid | 11.0 |
| Magnesium chloride | 5.0 |
| Sodium bicarbonate | 10.0 |
| Sodium fluoride | 2.0 |
| Flavor | 4.0 |
| Saccharin | 2.0 |
| Usage concentration | 5.0 grams/ounce |

| | |
|---|---|
| *Calcium nitrate encapsulated with water-soluble ethyl cellulose encapsulant. | |

### Examples 18-20

Examples 18-20 illustrate various embodiments of the present invention. Examples 18-20 represent both sugarless (Examples 18 and 19) and chewing gum containing sugar (Example 20). All contain divalent metal salts as stabilizers.

| | | | |
|---|---|---|---|
| Calcium lactate | 7.0 | -- | 1.5 |
| Calcium acetate | -- | 4.0 | 3.5 |
| Monopotassium phosphate | 2.8 | 3.3 | 4.0 |
| Dipotassium phosphate | 0.3 | 0.2 | 0.4 |
| Magnesium oxide | 0.2 | 0.3 | 0.3 |
| Gum Base | 25.0 | 30.0 | 20.0 |
| Sugar | -- | -- | 58.5 |
| Glucose | -- | -- | 10.0 |
| Sorbitol Powder | 34.6 | 54.85 | -- |
| Mannitol Powder | 15.0 | -- | -- |
| Maltitol Powder | 10.0 | -- | -- |
| Flavor | 1.5 | 1.3 | 1.8 |
| Glycerine | 3.5 | 6.0 | -- |
| Saccharin | 0.1 | -- | -- |
| Aspartame | -- | 0.05 | -- |

### Examples 21-23

Examples 21-23 illustrate further embodiments of the present invention.

Example 21 illustrates an embodiment where the calcium salt is encapsulated with a hydrophilic polymer; no divalent metal salt stabilizer is used.

Example 22 illustrates an embodiment where the calcium salt is encapsulated with a hydrophobic polymer; a divalent metal salt stabilizer is used.

Example 23 illustrates an embodiment where a desiccant is used in the anhydrous chewing gum product; no encapsulation or divalent metal salts are used.

| | Example No. | | |
|---|---|---|---|
| | 21 | 22 | 23 |
| Calcium lactate | 7.0 | -- | -- |
| (Hydrophilic polymer | | | |
| encapsulated) | | | |
| Calcium acetate | -- | -- | 5.0 |
| Calcium acetate | -- | 4.0 | -- |
| (Hydrophobic polymer | | | |
| encapsulated) | | | |
| Monopotassium phosphate | 2.8 | 3.3 | 4.0 |
| Dipotassium phosphate | 0.3 | 0.2 | 0.4 |
| Magnesium oxide | -- | 0.3 | -- |
| Anhydrous magnesium | -- | -- | 1.0 |
| chloride (desiccant) | | | |
| Gum base | 25.0 | 30.00 | 20.0 |
| Sugar | -- | -- | 57.8 |
| Glucose | -- | -- | 10.0 |
| Sorbitol (solution 70%) | -- | 15.0 | -- |
| Sorbitol powder | 34.8 | 39.85 | -- |
| Mannitol powder | 15.0 | -- | -- |
| Maltitol powder | 10.0 | -- | -- |
| Flavor | 1.5 | 1.3 | 1.8 |
| Glycerine | 3.5 | 6.0 | -- |
| Saccharin | 0.1 | -- | -- |
| Aspartame | -- | 0.05 | -- |

### Examples 24 and 25

An additional study was performed using the formulations below in order to illustrate remineralization hardness. The regimen for these tests was similar to the previous tests followed in Examples 1-4. However, in Examples 24 and 25, half of each chip was covered with tape to serve as an untreated control.

| | Example No. | |
|---|---|---|
| | 24 | 25 |
| Part A | | |
| Calcium nitrate | 4.6 | 6.7 |
| Magnesium chloride | 0.8 | -- |
| Stannous chloride | -- | 0.04 |
| Water | 94.6 | 93.26 |

| Part B | | |
|---|---|---|
| Dipotassium phosphate | 0.38 | 0.5 |
| Monopotassium phosphate | 2.32 | 3.4 |
| Water | 97.3 | 96.1 |
| Hardness | 5.7 | 7.8 |

Three of the specimens from Example 24 and four specimens from Example 25 were sliced across the tape to expose both the treated and untreated portions of the lesion. SEM photomicrographs of each specimen were then prepared.

Examination of the SEM photomicrographs shows the presence of remineralization in six of the seven specimens examined as shown by a reduction in the holes and fissures in the treated sides of the specimens.

## Claims

1. A product for effecting remineralisation of one or more lesions formed in at least one subsurface of at least one tooth and/or mineralization of one or more tubules in exposed dentin in at least one tooth, the product comprising:
(i) from 0.05% to 15.0% by weight of at least one water-soluble calcium salt;
(ii) 0.001% to 2.0% by weight of at least one water-soluble, non-toxic salt of a divalent metal wherein the divalent metal is other than calcium;
(iii) from 0.05% to 15.0% by weight-of at least one water-soluble phosphate salt;
(iv) from 0% to 5.0% by weight of at least one water-soluble fluoride salt;
wherein, when ingredients (i)-(iv) are mixed together to form an aqueous mixed solution, the solution has a pH in the range of from 4.5 to 7.0.

2. A product as claimed in claim 1, wherein the pH of the aqueous solution is in the range of from 5.0 to 7.0.

3. A product as claimed in claim 2, wherein the pH of the aqueous solution is in the range of from 5.0 to 5.75.

4. A product as claimed in claim 1, 2 or 3, wherein the divalent metal is magnesium, strontium, tin or zinc.

5. A product as claimed in any preceding claim, wherein the divalent metal salt is magnesium chloride, magnesium acetate, magnesium oxide, strontium acetate, strontium chloride, strontium nitrate, stannous chloride, zinc acetate, zinc chloride, zinc sulphate, zinc nitrate, or a mixture thereof.

6. A product as claimed in any preceding claim, wherein the at least one fluoride salt is present in an amount in the range of from 0.01% to 5% by weight.

7. A product as claimed in any preceding claim, wherein the aqueous solution consists essentially of from 100 ppm to 35,000 ppm of calcium ions released by the at least one calcium salt, from 100 ppm to 40,000 ppm of phosphate ions released by the at least one phosphate salt, from 10 ppm to 20,000 ppm of divalent metal ions released by the at least one divalent metal salt, and from 20 ppm to 5000 ppm fluoride ions released by the at least one fluoride salt.

8. A product as claimed in any preceding claim, which is an aqueous, stable, one-part product.

9. A product as claimed in claim 8, which is a toothpaste, a gel, a professional gel, a dental cream, a mouthwash, a mouthrinse, a chewing gum, a lozenge, a food product, a dragee or a bon bon.

10. A product as claimed in any one of claims 1 to 7, which is a non-aqueous, stable one-part product and which further comprises:
(v) a stabilizing agent selected from the group consisting of up to 7.5% by weight of a desiccating agent and an encapsulating coating on particles of at least one of ingredients (i) and (iii) wherein the coating is capable of readily dissolving, dispersing or emulsifying in saliva.

11. A product as claimed in any one of claims 1 to 7, which is an aqueous, stable two-part product and comprises:
(a) a first discrete part comprising the at least one water-soluble calcium salt and the at least one non-toxic, water-soluble divalent metal salt other than calcium salt; and
(b) a second discrete part comprising the at least one water-soluble phosphate salt and the at least one water-soluble fluoride salt; and
(c) a dispensing means for simultaneously dispensing the first and second parts.

12. A product as claimed in claim 11, wherein the first and second parts and the dispensing means are disposed in a dispensing container, the dispensing container comprising:
a first compartment having a first outlet and a second compartment having a second outlet, the first compartment storing the first part and the second compartment storing the second part, the first outlet and the second outlet being disposed in proximity to one another;
a closure mechanism for closing the first and second compartments; and
a dispensing mechanism for simultaneously dispensing the first and second parts through the first and second outlets, respectively, onto the surface.

13. A product as claimed in claim 11 or claim 12, wherein the first and second parts are independently selected from the group consisting of a toothpaste, a gel, a professional gel, a dental cream, a mouthwash and a mouthrinse.

14. A product as claimed in any one of claims 1-7, which is a chewing gum product and which further comprises:
(vi) from 10.0% to 95.0% by weight of a gum base.

15. A product as claimed in claim 14, further comprising (vii) a stabilizing agent selected from the group consisting of up to 7.5% by weight of a desiccating agent and an encapsulating coating on particles of at least one of ingredients (i) and (iii) wherein the coating is capable of readily dissolving, dispersing or emulsifying in saliva.

16. A product according to claim 8 or claim 9 for use in a method of remineralising one or more lesions disposed in at least one subsurface of at least one tooth and/or mineralizing one or more tubules in exposed dentin in the at least one tooth, comprising:
contacting the at least one tooth with said product for a sufficient period of time that calcium ions released from the at least one calcium salt and phosphate ions released from the at least one phosphate salt diffuse into the at least one subsurface of the at least one tooth and precipitate to form hydroxyapatite at the at least one subsurface and thereby remineralise the one or more lesions and/or mineralize the one or more tubules.

17. A product according to claim 10 for use in a method of remineralising one or more lesions formed in at least one subsurface of at least one tooth and/or mineralizing one or more tubules in exposed dentin in the at least one tooth, comprising:
dissolving said product in water and/or in saliva to form an aqueous mixed solution; and
contacting the at least one tooth with the aqueous mixed solution for a sufficient period of time that calcium ions released from the at least one calcium salt and phosphate ions released from the at least one phosphate salt diffuse into the at least one subsurface of the at least one tooth and precipitate to form hydroxyapatite at the at least one subsurface and thereby remineralise the one or more lesions and/or mineralize the one or more tubules.

18. A product according to any one of claims 11, 12 or 13 for use in a method of remineralising one or more lesions formed in at least one subsurface of at least one tooth and/or mineralizing one or more tubules in exposed dentin in the at least one tooth, comprising:
simultaneously dispensing the first and second parts of said product;
mixing the simultaneously dispensed first and second parts in the oral cavity to form an aqueous mixed solution; and
contacting the at least one tooth with the aqueous mixed solution for a sufficient period of time that calcium ions released from the at least one calcium salt and phosphate ions released from the at least one phosphate salt diffuse into the at least one subsurface of the at least one tooth and precipitate to form hydroxyapatite at the at least one subsurface and thereby remineralise the one or more lesions and/or mineralize the one or more tubules.

19. A product according to claim 14 or claim 15 for use in a method for remineralising one or more lesions in at least one subsurface of at least one tooth disposed in an oral cavity and/or mineralizing one or more tubules in exposed dentin in at least one tooth, comprising:
chewing the said product in the oral cavity so as to form the mixed aqueous solution, the chewing being carried out for a sufficient period of time that calcium ions released from the at least one calcium salt and phosphate ions released from the at least one phosphate salt diffuse into the at least one subsurface of the at least one tooth and precipitate to form hydroxyapatite at the at least one subsurface and thereby remineralise the one or more lesions and/or mineralize the one or more tubules.

## Patentansprüche

1. Erzeugnis für die Durchführung einer Remineralisierung von ein oder mehreren Läsionen, die sich in mindestens einer Unteroberfläche von mindestens einem Zahn ausgebildet haben und/oder einer Mineralisierung von ein oder mehreren Kanälen im freiliegenden Dentin von mindestens einem Zahn, umfassend
(i) 0,05 bis 15,0 Gew.% mindestens eines wasserlöslichen Calciumsalzes;
(ii) 0,001 bis 2,0 Gew.% mindestens eines wasserlöslichen, nicht-toxischen Salzes eines zweiwertigen Metalls, wobei das zweiwertige Metall nicht Calcium ist;
(iii)0,05 bis 15,0 Gew.% mindestens eines wasserlöslichen Phosphatsalzes;
(iv) 0 bis 5,0 Gew.% mindestens eines wasserlöslichen Fluoridsalzes;
wobei die Lösung einen pH im Bereich von 4,5 bis 7,0 besitzt, wenn die Zutaten (i) - (iv) zu einem wässrigen Lösungsgemisch zusammengemischt sind.

2. Erzeugnis nach Anspruch 1, wobei der pH der wässrigen Lösung 5,0 bis 7,0 ist.

3. Erzeugnis nach Anspruch 2, wobei der pH der wässrigen Lösung 5,0 bis 5,75 ist.

4. Erzeugnis nach Anspruch 1, 2 oder 3, wobei das zweiwertige Metall Magnesium, Strontium, Zinn oder Zink ist.

5. Erzeugnis nach irgendeinem vorhergehenden Anspruch, wobei das divalente Metallsalz Magnesiumchlorid ist, Magnesiumacetat, Magnesiumoxid, Strontiumacetat, Strontiumchlorid, Strontiumnitrat, Zinnchlorid, Zinkacetat, Zinkchlorid, Zinksulfat, Zinknitrat oder Gemische hiervon.

6. Erzeugnis nach irgendeinem vorhergehenden Anspruch, wobei das mindestens eine Fluoridsalz in einer Menge von 0,01 bis 5 Gew.% zugegen ist.

7. Erzeugnis nach irgendeinem vorhergehenden Anspruch, wobei die wässrige Lösung im Wesentlichen besteht aus 100 ppm bis 35000 ppm Calciumionen, die von mindestens einem Calciumsalz freigesetzt werden, 100 ppm bis 40000 ppm Phosphationen, die von mindestens einem Phosphatsalz freigesetzt werden, 10 bis 20000 ppm zweiwertige Metallionen, die von mindestens einem zweiwertigen Metallsalz freigesetzt werden und 20 bis 5000 ppm Fluoridionen, die von mindestens einem Fluoridsalz freigesetzt werden.

8. Erzeugnis nach irgendeinem vorhergehenden Anspruch, das ein wässriges stabiles einteiliges Produkt ist.

9. Erzeugnis nach Anspruch 8, welche eine Zahnpasta ist, ein Gel, ein professionelles Gel, eine Zahncreme, ein Mundwaschmittel, ein Mundspülmittel, ein Kaugummi, ein Gurgelmittel, ein Nahrungserzeugnis, ein Dragee oder ein Bonbon.

10. Erzeugnis nach irgendeinem der Ansprüche 1 bis 7, das ein nicht-wässriges stabiles einteiliges Produkt ist und zudem enthält
(v) ein Stabilisierungsmittel, ausgewählt aus der Gruppe, bestehend aus bis zu 7,5 Gew.% Trockenmittel und einer Kapselbeschichtung auf Teilchen mit mindestens einer der Zutaten (i) bis (iii, wobei sich die Beschichtung bei einem Dispergieren oder Emulgieren im Speichel sofort auflöst.

11. Erzeugnis nach irgendeinem der Ansprüche 1 bis 7, das ein wässriges, stabiles zweiteiliges Produkt ist und enthält
(a) einen ersten diskreten Anteil, enthaltend mindestens ein wasserlösliches Calciumsalz und mindestens ein nicht-toxisches wasserlösliches divalentes Metallsalz, das kein Calciumsalz ist und
(b) einen zweiten diskreten Anteil, umfassend das mindestens eine wasserlösliche Phosphatsalz und das mindestens eine wasserlösliche Fluoridsalz; und
(c) ein Dispergierungsmittel zum gleichzeitigen Dispergieren des ersten und des zweiten Anteils.

12. Erzeugnis nach Anspruch 11, wobei der erste und der zweite Anteil sowie das Dispergierungsmittel in einem Abgabebehältnis angeordnet sind, wobei das Abgabebehältnis umfasst
eine erste Kammer mit einem ersten Auslass und eine zweite Kammer mit einem zweiten Auslass, wobei die erste Kammer den ersten Anteil und die zweite Kammer den zweiten Anteil enthält und der erste Auslass und der zweite Auslass in Nachbarschaft zueinander angeordnet sind;
einen Verschlussmechanismus zum Verschließen des ersten und der zweiten Kammer; und
einen Abgabemechanismus zum zeitgleichen Abgeben des ersten und des zweiten Anteils durch den ersten bzw. den zweiten Auslass auf die Oberfläche.

13. Erzeugnis nach Anspruch 11 oder Anspruch 12, wobei der erste und der zweite Teil unabhängig ausgewählt sind aus der Gruppe Zahnpasta, Gel, professionelles Gel, Zahncreme, Mundwaschmittel und Mundspülmittel.

14. Erzeugnis nach irgendeinem der Ansprüche 1 bis 7, das ein Kaugummiprodukt ist und zudem (vi) 10,0 bis 95,0 Gew.% Gummibasis aufweist.

15. Erzeugnis nach Anspruch 14, das zudem (vii) ein Stabilisierungsmittel aufweist, ausgewählt aus der Gruppe mit bis zu 7,5 Gew.% Trockenmittel und einer Kapselbeschichtung auf Teilchen mit mindestens einer der Zutaten (i) und (iii), wobei die Beschichtung sich bei einem Dispergieren oder Emulgieren im Speichel sofort auflöst.

16. Ein Erzeugnis nach Anspruch 8 oder Anspruch 9 zur Verwendung in einem Verfahren zur Remineralisierung ein oder mehrerer Läsionen, die sich in mindestens einer Unteroberfläche von mindestens einem Zahn befinden und/oder für die Mineralisierung ein oder mehrerer Kanäle im freiliegendem Dentin in dem mindestens einen Zahn, umfassend
das Zusammenbringen des mindestens einen Zahns mit dem Erzeugnis für eine solange Zeitdauer, dass Calciumionen des mindestens einen Calciumsalzes und Phosphationen des mindestens einen Phosphatsalzes freigesetzt werden und in die mindestens eine Unteroberfläche des mindestens einen Zahns diffundieren und unter Bildung von Hydroxyapatit an der mindestens einen Unteroberfläche präzipitieren und hierdurch die ein oder mehreren Läsionen remineralisieren und/oder die ein oder mehreren Kanäle mineralisieren.

17. Erzeugnis nach Anspruch 10 zur Verwendung in einem Verfahren zur Remineralisierung ein oder mehrerer Läsionen, die in mindestens einer Unteroberfläche von mindestens einem Zahn sich befinden und/oder zur Mineralisierung ein oder mehrerer Kanäle im freiliegenden Dentin des mindestens einen Zahns, umfassend
das Auflösen des Produkts in Wasser und/oder in Speichel unter Bildung eines wässrigen Lösungsgemisches; und
das Zusammenbringen des mindestens einen Zahns mit dem wässrigen Lösungsgemisch für eine so lange Zeitdauer, dass Calciumionen aus dem mindestens einen Calciumsalz und Phosphationen aus dem mindestens einem Phosphatsalz freigesetzt werden und in die mindestens eine Unteroberfläche des mindestens einen Zahns diffundieren und unter Bildung von Hydroxyapatit an der mindestens einen Unteroberfläche präzipitieren und so die ein oder mehreren Läsionen remineralisieren und/oder die ein oder mehreren Kanäle mineralisieren.

18. Erzeugnis nach irgendeinem der Ansprüche 11, 12 oder 13 zur Verwendung in einem Verfahren zur Remineralisierung ein oder mehrerer Läsionen, die sich in mindestens einer Unteroberfläche von mindestens einem Zahn gebildet haben, und/oder zur Mineralisierung von ein oder mehreren Kanälen im freiliegenden Dentin des mindestens einen Zahns, umfassend
das zeitgleiche Verteilen des ersten und des zweiten Anteils des Produkts, das Mischen der gleichzeitig verteilten ersten und zweiten Anteile im Mundraum unter Bildung eines wässrigen Lösungsgemisches; und
das Zusammenbringen des mindestens einen Zahns mit dem wässrigen Lösungsgemisch für eine so lange Zeitdauer, dass Calciumionen freigesetzt werden aus dem mindestens einem Calciumsalz und Phosphationen aus dem mindestens einen Phosphatsalz und in die mindestens eine Unteroberfläche des mindestens einen Zahns diffundieren und unter Bildung von Hydroxyapatit an der mindestens einen Unteroberfläche präzipitieren und so die ein oder mehreren Läsionen remineralisieren und/oder die ein oder mehreren Kanäle mineralisieren.

19. Erzeugnis nach Anspruch 14 oder 15 zur Verwendung in einem Verfahren zur Remineralisierung ein oder mehrerer Läsionen in der mindestens einen Unteroberfläche des mindestens einen Zahns, der sich im Mundraum befindet und/oder zur Mineralisierung ein oder mehrerer Kanäle im freiliegenden Dentin des mindestens einen Zahns, umfassend
das Kauen des Erzeugnisses im Mundraum, so dass ein wässriges Lösungsgemisch gebildet wird, wobei das Kauen erfolgt für eine so lange Zeitdauer, dass Calciumionen freigesetzt werden aus dem mindestens einem Calciumsalz und Phosphationen aus dem mindestens einem Phosphatsalz und in die mindestens eine Unteroberfläche des mindestens einen Zahns diffundieren und unter Bildung von Hydroxyapatit präzipitieren an der mindestens einen Unteroberfläche und hierdurch die ein oder mehreren Läsionen remineralisieren und/oder die ein oder mehreren Kanäle mineralisieren.

## Revendications

1. Produit pour effectuer une reminéralisation d'une ou plusieurs lésions formées sur au moins une subsurface d'au moins une dent et/ou une minéralisation d'un ou plusieurs canalicules dans la dentine exposée dans au moins une dent, le produit comprenant :
(i) de 0,05% à 15,0% en masse d'au moins un sel de calcium soluble dans l'eau ;
(ii) de 0,001 % à 2,0% en masse d'au moins un sel soluble dans l'eau, non toxique d'un métal divalent dans lequel le métal divalent est différent du calcium ;
(iii) de 0,05% à 15,0% en masse d'au moins un sel phosphate soluble dans l'eau ;
(iv) de 0% à 5,0% en masse d'au moins un sel fluorure soluble dans l'eau ;
dans lequel lorsque les ingrédients (i) à (iv) sont mélangés ensemble pour former une solution aqueuse mixte, la solution présente un pH dans l'intervalle de 4,5 à 7,0.

2. Produit tel que revendiqué dans la revendication 1, dans lequel le pH de la solution aqueuse est dans l'intervalle de 5,0 à 7,0.

3. Produit tel que revendiqué dans la revendication 2, dans lequel le pH de la solution aqueuse est dans l'intervalle de 5,0 à 5,75.

4. Produit tel que revendiqué dans la revendication 1, 2 ou 3, dans lequel le métal divalent est le magnésium, le strontium, l'étain ou le zinc.

5. Produit tel que revendiqué dans l'une quelconque des revendications précédentes, dans lequel le sel d'un métal divalent est le chlorure de magnésium, l'acétate de magnésium, l'oxyde de magnésium, l'acétate de strontium, le chlorure de strontium, le nitrate de strontium, le chlorure stanneux, l'acétate de zinc, le chlorure de zinc, le sulfate de zinc, le nitrate de zinc ou un de leurs mélanges.

6. Produit tel que revendiqué dans l'une quelconque des revendications précédentes, dans lequel le au moins un sel fluorure est présent en une quantité dans l'intervalle de 0,01% à 5% en masse.

7. Produit tel que revendiqué dans l'une quelconque des revendications précédentes, dans lequel la solution aqueuse est constituée essentiellement de 100 ppm à 35 000 ppm d'ions calcium libérés par le au moins un sel de calcium, de 100 ppm à 40 000 ppm d'ions phosphate libérés par le au moins un sel phosphate, de 10 ppm à 20 000 ppm d'ions d'un métal divalent libérés par le au moins un sel d'un métal divalent et de 20 ppm à 5000 ppm d'ions fluorure libérés par le au moins un sel fluorure.

8. Produit tel que revendiqué dans l'une quelconque des revendications précédentes, qui est un produit aqueux, stable à un seul composant.

9. Produit tel que revendiqué dans la revendication 8, qui est une pâte dentifrice, un gel, un gel professionnel, une crème dentaire, un bain de bouche, un produit pour rincer la bouche, une gomme à mâcher, une pastille, un produit alimentaire, une dragée ou un bonbon.

10. Produit tel que revendiqué dans l'une quelconque des revendications 1 à 7, qui est un produit non aqueux, stable à un seul composant et qui comprend en outre :
(v) un agent stabilisant choisi dans le groupe constitué par jusqu'à 7,5% en masse d'un agent de dessiccation et d'un revêtement d'encapsulation sur les particules d'au moins un des ingrédients (i) et (iii), dans lequel le revêtement est capable de se dissoudre, de se disperser ou de s'émulsifier facilement dans la salive.

11. Produit tel que revendiqué dans l'une quelconque des revendications 1 à 7, qui est un produit aqueux, stable à deux composants et qui comprend :
(a) un premier composant séparé comprenant le au moins un sel de calcium soluble dans l'eau et le au moins un sel soluble dans l'eau, non toxique d'un métal divalent autre qu'un sel de calcium ; et
(b) un deuxième composant séparé comprenant le au moins un sel phosphate soluble dans l'eau et le au moins un sel fluorure soluble dans l'eau ; et
(c) des moyens de délivrance pour délivrer simultanément les premier et deuxième composants.

12. Produit tel que revendiqué dans la revendication 11, dans lequel les premier et deuxième composants et les moyens de délivrance sont placés dans un récipient distributeur, le récipient distributeur comprenant :
un premier compartiment comportant un premier orifice de sortie et un deuxième compartiment comportant un deuxième orifice de sortie, le premier compartiment stockant le premier composant et le deuxième compartiment stockant le deuxième composant, le premier orifice de sortie et le deuxième orifice de sortie étant placés à proximité l'un de l'autre ;
un mécanisme de fermeture pour fermer les premier et deuxième compartiments ; et
un mécanisme distributeur pour délivrer à la surface simultanément les premier et deuxième composants à travers respectivement les premier et deuxième orifices de sortie.

13. Produit tel que revendiqué dans la revendication 11 ou la revendication 12, dans lequel les premier et deuxième composants sont choisis indépendamment dans le groupe constitué par une pâte dentifrice, un gel, un gel professionnel, une crème dentaire, un bain de bouche et un produit pour rincer la bouche.

14. Produit tel que revendiqué dans l'une quelconque des revendications 1 à 7, qui est un produit de gomme à mâcher et qui comprend en outre :
(vi) de 10,0% à 95,0% en masse d'une base de gomme.

15. Produit tel que revendiqué dans la revendication 14, comprenant en outre (vii) un agent stabilisant choisi dans le groupe constitué par jusqu'à 7,5% en masse d'un agent de dessiccation et d'un revêtement d'encapsulation sur les particules d'au moins un des ingrédients (i) et (iii), dans lequel le revêtement est capable de se dissoudre, de se disperser ou de s'émulsifier facilement dans la salive.

16. Produit selon la revendication 8 ou la revendication 9 pour une utilisation dans une méthode de reminéralisation d'une ou plusieurs lésions formées sur au moins une subsurface d'au moins une dent et/ou de minéralisation d'un ou plusieurs canalicules dans la dentine exposée dans la au moins une dent, comprenant :
la mise en contact de la au moins une dent avec ledit produit pendant une période de temps suffisante pour que les ions calcium libérés à partir du au moins un sel de calcium et les ions phosphate libérés à partir du au moins un sel phosphate diffusent dans la au moins une subsurface de la au moins une dent et précipitent pour former de l'hydroxyapatite sur la au moins une subsurface et ainsi reminéralisent la une ou plusieurs lésions et/ou minéralisent le un ou plusieurs canalicules.

17. Produit selon la revendication 10 pour une utilisation dans une méthode de reminéralisation d'une ou plusieurs lésions formées sur au moins une subsurface d'au moins une dent et/ou de minéralisation d'un ou plusieurs canalicules dans la dentine exposée dans la au moins une dent, comprenant :
la dissolution dudit produit dans l'eau et/ou la salive pour former une solution aqueuse mixte ; et
la mise en contact de la au moins une dent avec la solution aqueuse mixte pendant une période de temps suffisante pour que les ions calcium libérés à partir du au moins un sel de calcium et les ions phosphate libérés à partir du au moins un sel phosphate diffusent dans la au moins une subsurface de la au moins une dent et précipitent pour former de l'hydroxyapatite sur la au moins une subsurface et ainsi reminéralisent la une ou plusieurs lésions et/ou minéralisent le un ou plusieurs canalicules.

18. Produit selon l'une quelconque des revendications 11, 12 ou 13 pour une utilisation dans une méthode de reminéralisation d'une ou plusieurs lésions formées sur au moins une subsurface d'au moins une dent et/ou de minéralisation d'un ou plusieurs canalicules dans la dentine exposée dans la au moins une dent, comprenant :
la délivrance simultanément des premier et deuxième composants dudit produit ;
le mélange des premier et deuxième composants délivrés simultanément dans la cavité orale pour former une solution aqueuse mixte ; et
la mise en contact de la au moins une dent avec la solution aqueuse mixte pendant une période de temps suffisante pour que les ions calcium libérés à partir du au moins un sel de calcium et les ions phosphate libérés à partir du au moins un sel phosphate diffusent dans la au moins une subsurface de la au moins une dent et précipitent pour former de l'hydroxyapatite sur la au moins une subsurface et ainsi reminéralisent la une ou plusieurs lésions et/ou minéralisent le un ou plusieurs canalicules.

19. Produit selon la revendication 14 ou la revendication 15 pour une utilisation dans une méthode de reminéralisation d'une ou plusieurs lésions sur au moins une subsurface d'au moins une dent disposée dans une cavité orale et/ou de minéralisation d'un ou plusieurs canalicules dans la dentine exposée dans au moins une dent, comprenant :
le mâchage dudit produit dans la cavité orale de manière à former la solution aqueuse mixte, le mâchage étant effectué pendant une période de temps suffisante pour que les ions calcium libérés à partir du au moins un sel de calcium et les ions phosphate libérés à partir du au moins un sel phosphate diffusent dans la au moins une subsurface de la au moins une dent et précipitent pour former de l'hydroxyapatite sur la au moins une subsurface et ainsi reminéralisent la une ou plusieurs lésions et/ou minéralisent le un ou plusieurs canalicules.
